# EUROPEAN PATENT APPLICATION

(11) **EP 4 516 923 A1**
(43) Date of publication of application: **05.03.2025**
(21) Application number: 23795551.3
(22) Date of filing: 27.04.2023
(51) Int. Cl.: C12Q 1/68, C12Q 1/6883, C07K 16/18

(54) **DIGESTED DDIT4L PRODUCT AS DIAGNOSTIC MARKER FOR ALZHEIMER'S DISEASE**

(30) Priority: 29.04.2022 WO PCT/CN2022/090220
(71) Applicant: Plasmarker Biotechnology Co., Ltd, Suzhou, Jiangsu 215123 (CN)
(72) Inventor: LI, Kaicheng, Suzhou, Jiangsu 215123 (CN); YOU, Pu, Suzhou, Jiangsu 215123 (CN)
(74) Representative: Weickmann & Weickmann PartmbB
(86) International application number: PCT/CN2023/091199
(87) International publication number: WO 2023/208119

(57) **Abstract**

The present application relates to a digested DDIT4L product as a diagnostic marker for Alzheimer's disease, and use thereof in diagnosing Alzheimer's disease. In particular, the present application relates to use of a substance for detecting a digested intron retention (DIR) product encoding DNA-damage-inducible transcript 4 like (DDIT4L) in a sample of a subject in preparing a product for diagnosing Alzheimer's disease or a mild cognitive disorder and/or assessing (e.g., grading or staging) cognitive disorder progression, a related product thereof, and a method for screening a medicament using the DIR product.

## Description

### FIELD OF THE INTVENTION

The present application relates to the field of biomedicine, and in particular to application of a DNA damage-inducible transcript 4-like (DDIT4L) intron retention spliced product (DIR) as a blood marker for presenile dementia or mild cognitive impairment, as well as a product and method corresponding thereto.

### BACKGROUND OF THE INVENTION

Presenile dementia, also known as Alzheimer's disease (AD), is a neurodegenerative disease with the highest incidence among diseases that harm the human brain. It causes great suffering to patients themselves, and also brings a heavy burden to their families and the society.

According to "World Alzheimer Report 2017" from the Alzheimer's Disease International (ADI), the cost of taking caring of patients with dementia across the world has increased from US$604 billion in 2010 to US$1,000 billion in 2017, with a growth rate of 65.6%. There are 5 million AD patients in the United States, and caregivers need to spend 18 billion hours a year to take care of them. In 2017, there were 50 million AD patients around the world, and this number will continue to increase with the increase of the world's aging population. In the context where no medicament is available for treating, delaying or stopping the progression of the disease at present, the number of patients is expected to double every 20 years, reaching 82 million in 2030 and 150 million in 2050.

According to "China Development Report 2020: Development Trends in and Policies of Population Aging in China" released by the China Development Foundation (a national social organization initiated by the Development Research Center of the State Council), elderly people aged 65 and above in China are to be about 180 million by 2020, accounting for about 13% of the total population; above 210 million by 2025 when the "14th Five-Year Plan" is completed, accounting for about 15% of the total population; and up to 310 million and nearly 380 million in 2035 and 2050, accounting for up to 22.3% and 27.9% of the total population, respectively.

The incidence rate of AD is age-related and ranges from 2% to 4% of the incidence of the whole population. In particular, for middle-aged and elderly people aged over 65 years old, the prevalence rate is 5% and increases exponentially with the increase of age. Based on analysis, the prevalence rate can increase to 25% for elderly people aged over 85 years old, and is as high as 60% for those over 95 years old. China has gradually entered an aging society. According to statistics, there are nearly 10 million AD patients in China, and more than 300,000 new cases emerge every year.

The main symptom of AD is cognitive functional impairment. As it turns out, the cognitive function impairment is caused by synaptic and neuronal damage, and abnormal neural circuit structure and function. At present, the main reasons for the continuous failure of clinical research on new drugs for AD lie in unclear mechanism of the synaptic and neuronal damage, and lack of cell/animal models approaching the pathophysiological characteristics of human diseases (such as neurodegeneration, etc.), leading to slow progress of researches on effective methods for preventing and treating AD, and on early diagnostic markers capable of reflecting disease prognosis.

Metabolic disorders may be one of the main pathogenesis of AD. In AD patients, abnormal lipid metabolism revealed by cholesterol or the like and abnormal glucose metabolism revealed by glucose or the like are the most common metabolic disorders, which also include energy metabolism disorders like cerebral ischemia and hypoxia caused by cerebral circulation insufficiency, as represented by degenerative changes in cerebral small blood vessels. However, how the abnormal lipid metabolism and the abnormal glucose metabolism in brain affect the occurrence and development course of AD are unclear in mechanism. Mitochondria are key organelles for the metabolism of main energy substances, and their dysfunction can lead to abnormal energy metabolism and also damage the functions of neurons and the endothelial cells of cerebral small blood vessels. However, in the incidence and development of early AD, the relationship between mitochondrial dysfunction and abnormal lipid metabolism and glucose metabolism is not clear.

Mild cognitive impairment (MCI), a syndrome involving objective cognitive decline, is a state between mild dementia and healthy aging, with memory impairment as the prominent manifestation. It may involve other cognitive declines to a certain degree and normal competence for daily activities, and thus does not meet the diagnostic criteria for dementia. 10% to 15% of MCI are converted into AD every year, which is 10 times that of normal controls. As a transitional state from normal aging to dementia, MCI has attracted more and more attention, and studying MCI is an important part in the early diagnosis and early intervention of AD.

Current diagnostic markers for AD mainly include detection of Aβ and Tau contents in cerebrospinal fluid, PET and MRI neuroimaging means, etc. Although these methods have higher or even very high diagnostic efficacy, they also have shortcomings such as difficulty in operation, high cost, and weak correlation between injury and disease outcome. Accordingly, there is an urgent need to develop a new marker that is simple, easy to implement, cost-effective, and suitable for large-scale population screening, and that can be better used for early diagnosis of AD.

### SUMMARY OF THE INVENTION

The present application provides a novel blood marker for Alzheimer's disease (AD) or mild cognitive impairment (MCI).

In one aspect, the present application provides use of a DNA damage-inducible transcript 4-like (DDIT4L) intron retention spliced product (DIR), and/or a nucleic acid encoding the DIR, in diagnosis of a disease and/or in evaluation of progression of the disease, wherein the disease comprises cognitive impairment.

In one aspect, the present application provides use of a DNA damage-inducible transcript 4-like (DDIT4L) intron retention spliced product (DIR), and/or a nucleic acid encoding the DIR, in diagnosis of a disease and/or in evaluation of progression of the disease, wherein the disease comprises a neurodegenerative disease.

In some embodiments, the DIR comprises the amino acid sequence as set forth in SEQ ID NO: 1.

In some embodiments, the nucleic acid encoding the DIR comprises a nucleotide sequence as set forth in SEQ ID NO: 8.

In some embodiments, the nucleic acid encoding the DIR comprises a nucleotide sequence as set forth in SEQ ID NO: 93.

In some embodiments, the cognitive impairment comprises cognitive impairment caused by normal aging, Lewy body dementia (LBD), frontotemporal dementia and/or vascular dementia.

In some embodiments, the cognitive impairment induces diseases comprising Alzheimer's disease, multi-infarct type, Parkinson's disease, AIDS and/or Creutzfeldt-Jakob disease (CJD).

In some embodiments, the cognitive impairment comprises mild cognitive impairment (MCI), intermediate cognitive impairment and late cognitive impairment.

In some embodiments, the cognitive impairment comprises multi-domain amnestic MCI (aMCI-m).

In some embodiments, the neurodegenerative disease comprises an acute neurodegenerative disease and a chronic neurodegenerative disease.

In some embodiments, the neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

In some embodiments, the neurodegenerative disease comprises Alzheimer's disease, Parkinson's disease, multiple sclerosis (MS), amyotrophic lateral sclerosis (ALS) and/or Huntington's disease (HD).

In some embodiments, the neurodegenerative disease comprises presenile dementia.

In some embodiments, the neurodegenerative disease comprises early presenile dementia, intermediate presenile dementia and/or late presenile dementia.

In another aspect, the present application provides use of a substance for detecting a DNA damage-inducible transcript 4-like DDIT4L intron retention spliced product DIR, and/or a nucleic acid encoding said DIR, in preparation of a product for diagnosis of a disease and/or in evaluation of progression of said disease, wherein said disease comprises cognitive impairment.

In another aspect, the present application provides use of a substance for detecting a DNA damage-inducible transcript 4-like (DDIT4L) intron retention spliced product (DIR), and/or a nucleic acid encoding the DIR, in preparation of a product for diagnosis of a disease and/or in evaluation of progression of the disease, wherein the disease comprises a neurodegenerative disease.

In another aspect, the present application provides a diagnostic kit, comprising the substance capable of detecting a DNA damage-inducible transcript 4-like (DDIT4L) intron retention spliced product (DIR), and/or a nucleic acid encoding the DIR, as described in the present application, wherein the diagnostic kit is for use in diagnosis of cognitive impairment, and/or evaluation of progression of the cognitive impairment; and/or for use in diagnosis of a neurodegenerative disease, and/or evaluation of progression of the neurodegenerative disease.

In some embodiments, the diagnostic kit comprises an instruction manual recording a method of using the diagnostic kit for diagnosis of cognitive impairment, and/or evaluation of progression of the cognitive impairment; and/or for diagnosis of a neurodegenerative disease, and/or evaluation of progression of the neurodegenerative disease.

In some embodiments, the DIR comprises the amino acid sequence as set forth in SEQ ID NO: 1.

In some embodiments, the nucleic acid encoding the DIR comprises a nucleotide sequence as set forth in SEQ ID NO: 8.

In some embodiments, the nucleic acid encoding the DIR comprises a nucleotide sequence as set forth in SEQ ID NO: 93.

In some embodiments, the cognitive impairment comprises cognitive impairment caused by normal aging, Lewy body dementia (LBD), frontotemporal dementia and/or vascular dementia.

In some embodiments, the cognitive impairment induces diseases comprising Alzheimer's disease, multi-infarct type, Parkinson's disease, AIDS and/or Creutzfeldt-Jakob disease (CJD).

In some embodiments, the cognitive impairment comprises mild cognitive impairment (MCI), intermediate cognitive impairment and late cognitive impairment.

In some embodiments, the cognitive impairment comprises multi-domain amnestic MCI (aMCI-m).

In some embodiments, the neurodegenerative disease comprises an acute neurodegenerative disease and a chronic neurodegenerative disease.

In some embodiments, the neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

In some embodiments, the neurodegenerative disease comprises Alzheimer's disease, Parkinson's disease, multiple sclerosis (MS), amyotrophic lateral sclerosis (ALS) and/or Huntington's disease (HD).

In some embodiments, the neurodegenerative disease comprises presenile dementia.

In some embodiments, the neurodegenerative disease comprises early presenile dementia, intermediate presenile dementia and/or late presenile dementia.

In some embodiments, the substance comprises a reagent and/or an apparatus capable of detecting the DIR, and/or the nucleic acid encoding the DIR.

In some embodiments, the substance comprises a reagent for specifically detecting the DIR, and/or the nucleic acid encoding the DIR.

In some embodiments, the substance comprises an antigen-binding protein capable of specifically binding to the DIR, a probe capable of specifically binding to the DIR, a primer capable of specifically amplifying the nucleic acid encoding the DIR, a gene chip capable of specifically detecting the nucleic acid encoding the DIR, an aptamer capable of specifically binding to the nucleic acid encoding the DIR, and/or a guide RNA capable of specifically binding to the nucleic acid encoding the DIR.

In some embodiments, the antigen-binding protein comprises LCDR2, which comprises the amino acid sequence as set forth in SEQ ID NO: 74.

In some embodiments, the LCDR2 comprises the amino acid sequence as set forth in SEQ ID NO: 26 or 16.

In some embodiments, the antigen-binding protein comprises LCDR1, which comprises the amino acid sequence as set forth in SEQ ID NO: 73.

In some embodiments, the LCDR1 comprises the amino acid sequence as set forth in any one of SEQ ID NOs: 25, 52 and 15.

In some embodiments, the antigen-binding protein comprises LCDR3, which comprises the amino acid sequence as set forth in SEQ ID NO: 75.

In some embodiments, the LCDR3 comprises the amino acid sequence as set forth in any one of SEQ ID NOs: 27, 53, 67 and 17.

In some embodiments, the antigen-binding protein comprises LCDR1, LCDR2, and LCDR3, wherein
a) the LCDR1 comprises the amino acid sequence as set forth in SEQ ID NO: 25, the LCDR2 comprises the amino acid sequence as set forth in SEQ ID NO: 26, and the LCDR3 comprises the amino acid sequence as set forth in SEQ ID NO: 27;
b) the LCDR1 comprises the amino acid sequence as set forth in SEQ ID NO: 52, the LCDR2 comprises the amino acid sequence as set forth in SEQ ID NO: 26, and the LCDR3 comprises the amino acid sequence as set forth in SEQ ID NO: 53;
c) the LCDR1 comprises the amino acid sequence as set forth in SEQ ID NO: 52, the LCDR2 comprises the amino acid sequence as set forth in SEQ ID NO: 26, and the LCDR3 comprises the amino acid sequence as set forth in SEQ ID NO: 67; or,
d) the LCDR1 comprises the amino acid sequence as set forth in SEQ ID NO: 15, the LCDR2 comprises the amino acid sequence as set forth in SEQ ID NO: 16, and the LCDR3 comprises the amino acid sequence as set forth in SEQ ID NO: 17.

In some embodiments, the antigen-binding protein comprises HCDR1, which comprises the amino acid sequence as set forth in SEQ ID NO: 70.

In some embodiments, the HCDR1 comprises the amino acid sequence as set forth in any one of SEQ ID NOs: 20, 35, 56 and 10.

In some embodiments, the antigen-binding protein comprises HCDR2, which comprises the amino acid sequence as set forth in SEQ ID NO: 71.

In some embodiments, the HCDR2 comprises the amino acid sequence as set forth in any one of SEQ ID NOs: 21, 36, 42, 48 and 11.

In some embodiments, the antigen-binding protein comprises HCDR3, which comprises the amino acid sequence as set forth in SEQ ID NO: 70.

In some embodiments, the HCDR3 comprises the amino acid sequence as set forth in any one of SEQ ID NOs: 22, 30, 37, 43, 49, 57, 62 and 12.

In some embodiments, the antigen-binding protein comprises HCDR1, HCDR2, and HCDR3, wherein
a) the HCDR1 comprises the amino acid sequence as set forth in SEQ ID NO: 20, the HCDR2 comprises the amino acid sequence as set forth in SEQ ID NO: 21, and the HCDR3 comprises the amino acid sequence as set forth in SEQ ID NO: 22;
b) the HCDR1 comprises the amino acid sequence as set forth in SEQ ID NO: 20, the HCDR2 comprises the amino acid sequence as set forth in SEQ ID NO: 21, and the HCDR3 comprises the amino acid sequence as set forth in SEQ ID NO: 30;
c) the HCDR1 comprises the amino acid sequence as set forth in SEQ ID NO: 35, the HCDR2 comprises the amino acid sequence as set forth in SEQ ID NO: 36, and the HCDR3 comprises the amino acid sequence as set forth in SEQ ID NO: 37;
d) the HCDR1 comprises the amino acid sequence as set forth in SEQ ID NO: 20, the HCDR2 comprises the amino acid sequence as set forth in SEQ ID NO: 42, and the HCDR3 comprises the amino acid sequence as set forth in SEQ ID NO: 43;
e) the HCDR1 comprises the amino acid sequence as set forth in SEQ ID NO: 20, the HCDR2 comprises the amino acid sequence as set forth in SEQ ID NO: 48, and the HCDR3 comprises the amino acid sequence as set forth in SEQ ID NO: 49;
f) the HCDR1 comprises the amino acid sequence as set forth in SEQ ID NO: 56, the HCDR2 comprises the amino acid sequence as set forth in SEQ ID NO: 36, and the HCDR3 comprises the amino acid sequence as set forth in SEQ ID NO: 57;
g) the HCDR1 comprises the amino acid sequence as set forth in SEQ ID NO: 35, the HCDR2 comprises the amino acid sequence as set forth in SEQ ID NO: 36, and the HCDR3 comprises the amino acid sequence as set forth in SEQ ID NO: 62;
h) the HCDR1 comprises the amino acid sequence as set forth in SEQ ID NO: 35, the HCDR2 comprises the amino acid sequence as set forth in SEQ ID NO: 42, and the HCDR3 comprises the amino acid sequence as set forth in SEQ ID NO: 37;
i) the HCDR1 comprises the amino acid sequence as set forth in SEQ ID NO: 56, the HCDR2 comprises the amino acid sequence as set forth in SEQ ID NO: 36, and the HCDR3 comprises the amino acid sequence as set forth in SEQ ID NO: 57; or
j) the HCDR1 comprises the amino acid sequence as set forth in SEQ ID NO: 10, the HCDR2 comprises the amino acid sequence as set forth in SEQ ID NO: 11, and the HCDR3 comprises the amino acid sequence as set forth in SEQ ID NO: 12.

In some embodiments, the antigen-binding protein comprises a heavy chain variable region VH, which comprises the amino acid sequence as set forth in any one of SEQ ID NOs: 13, 23, 31, 38, 44, 50, 58, 63 and 65.

In some embodiments, the antigen-binding protein comprises a light chain variable region VL, which comprises the amino acid sequence as set forth in any one of SEQ ID NOs: 18, 28, 33, 40, 46, 54, 60 and 68.

In some embodiments, the antigen-binding protein comprises a heavy chain variable region VH and a light chain variable region VL, wherein:
a) the VH comprises the amino acid sequence as set forth in SEQ ID NO: 23 and the VL comprises the amino acid sequence as set forth in SEQ ID NO: 28;
b) the VH comprises the amino acid sequence as set forth in SEQ ID NO: 31 and the VL comprises the amino acid sequence as set forth in SEQ ID NO: 33;
c) the VH comprises the amino acid sequence as set forth in SEQ ID NO: 38 and the VL comprises the amino acid sequence as set forth in SEQ ID NO: 40;
d) the VH comprises the amino acid sequence as set forth in SEQ ID NO: 44 and the VL comprises the amino acid sequence as set forth in SEQ ID NO: 46;
e) the VH comprises the amino acid sequence as set forth in SEQ ID NO: 50 and the VL comprises the amino acid sequence as set forth in SEQ ID NO: 54;
f) the VH comprises the amino acid sequence as set forth in SEQ ID NO: 58 and the VL comprises the amino acid sequence as set forth in SEQ ID NO: 60;
g) the VH comprises the amino acid sequence as set forth in SEQ ID NO: 63 and the VL comprises the amino acid sequence as set forth in SEQ ID NO: 60;
h) the VH comprises the amino acid sequence as set forth in SEQ ID NO: 65 and the VL comprises the amino acid sequence as set forth in SEQ ID NO: 54;
i) the VH comprises the amino acid sequence as set forth in SEQ ID NO: 58 and the VL comprises the amino acid sequence as set forth in SEQ ID NO: 68; or
j) the VH comprises the amino acid sequence as set forth in SEQ ID NO: 13, and the VL comprises the amino acid sequence as set forth in SEQ ID NO: 18.

In some embodiments, the antigen-binding protein comprises a heavy chain constant region, which comprises an IgG-derived constant region.

In some embodiments, the heavy chain constant region comprises a constant region derived from a protein selected from the group consisting of: IgG1, IgG2, IgG3 and IgG4.

In some embodiments, the antigen-binding protein comprises a light chain constant region, which comprises an Igκ-derived constant region or an Igλ-derived constant region.

In some embodiments, the light chain constant region comprises a human Igκ-derived constant region.

In some embodiments, the antigen-binding protein comprises an antibody or an antigen-binding fragment thereof.

In some embodiments, the antigen-binding fragment is selected from the group consisting of: Fab, Fab', F(ab)2, an Fv fragment, F(ab')2, scFv, di-scFv, VHH and/or dAb.

In some embodiments, the antibody is selected from the group consisting of: a monoclonal antibody, a murine antibody and a chimeric antibody.

In some embodiments, the product comprises a diagnostic kit.

In another aspect, the present application provides a method for diagnosing cognitive impairment, and/or evaluating progression of the cognitive impairment, comprising the step of: detecting, from a sample of a subject in need thereof, a content of a DNA damage-inducible transcript 4-like (DDIT4L) intron retention spliced product (DIR), and/or of a nucleic acid encoding the DIR.

In another aspect, the present application provides a method for diagnosing a neurodegenerative disease, and/or evaluating progression of the neurodegenerative disease, comprising the step of: detecting, from a sample of a subject in need thereof, a content of a DNA damage-inducible transcript 4-like (DDIT4L) intron retention spliced product (DIR), and/or of a nucleic acid encoding the DIR.

In some embodiments, the DIR comprises the amino acid sequence as set forth in SEQ ID NO: 1.

In some embodiments, the nucleic acid encoding the DIR comprises a nucleotide sequence as set forth in SEQ ID NO: 8.

In some embodiments, the nucleic acid encoding the DIR comprises a nucleotide sequence as set forth in SEQ ID NO: 93.

In some embodiments, the cognitive impairment comprises cognitive impairment caused by normal aging, Lewy body dementia (LBD), frontotemporal dementia and/or vascular dementia.

In some embodiments, the cognitive impairment-inducing diseases comprise Alzheimer's disease, multi-infarct type, Parkinson's disease, AIDS and/or Creutzfeldt-Jakob disease (CJD).

In some embodiments, the cognitive impairment comprises mild cognitive impairment (MCI), intermediate cognitive impairment and late cognitive impairment.

In some embodiments, the cognitive impairment comprises multi-domain amnestic MCI (aMCI-m).

In some embodiments, the neurodegenerative disease comprises an acute neurodegenerative disease and a chronic neurodegenerative disease.

In some embodiments, the neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

In some embodiments, the neurodegenerative disease comprises Alzheimer's disease, Parkinson's disease, multiple sclerosis (MS), amyotrophic lateral sclerosis (ALS) and/or Huntington's disease (HD).

In some embodiments, the neurodegenerative disease comprises presenile dementia.

In some embodiments, the neurodegenerative disease comprises early presenile dementia, intermediate presenile dementia and/or late presenile dementia.

In some embodiments, the subject comprises a mammal.

In some embodiments, the subject comprises a tumor patient.

In some embodiments, the subject is in an old age stage.

In some embodiments, the sample comprises a blood sample and/or a tissue or cell sample.

In some embodiments, the sample comprises whole blood, serum, plasma, and/or cerebrospinal fluid.

In some embodiments, the method comprises: comparing the content of the DIR and/or of the nucleic acid encoding the DIR in the sample of the subject with a normal control value, wherein the normal control value comprises a content of the DIR and/or of the nucleic acid encoding the DIR in a normal subject.

In some embodiments, the normal subject does not suffer from the cognitive impairment and/or the neurodegenerative disease.

In some embodiments, when the content of the DIR and/or of the nucleic acid encoding the DIR in the sample of the subject is significantly higher than the normal control value, the subject is diagnosed with the cognitive impairment and/or the neurodegenerative disease.

In some embodiments, the method comprises: comparing the content of the DIR and/or of the nucleic acid encoding the DIR in the sample of the subject with an early control value, wherein the early control value comprises a previously measured content of the DIR and/or of the nucleic acid encoding the DIR in the same subject.

In some embodiments, the subject has been diagnosed with the cognitive impairment and/or the neurodegenerative disease.

In some embodiments, when the content of the DIR and/or of the nucleic acid encoding the DIR in the sample of the subject is significantly higher than the early control value, the subject is diagnosed with aggravated progression of the cognitive impairment and/or the neurodegenerative disease.

In some embodiments, the method further comprises the step of detecting a content of a marker associated with the cognitive impairment and/or the neurodegenerative disease in a sample derived from the subject.

In some embodiments, the marker associated with the cognitive impairment and/or the neurodegenerative disease comprises AD7C-NTP, pTau-181, pTau-217 and /or Aβ1-42.

In some embodiments, the method further comprises the step of observing brain imaging of the subject.

In another aspect, the present application provides a system for diagnosing cognitive impairment, and/or evaluating progression of the cognitive impairment, comprising a substance for detecting a DNA damage-inducible transcript 4-like (DDIT4L) intron retention spliced product (DIR), and/or a nucleic acid encoding the DIR.

In another aspect, the present application provides a system for diagnosing a neurodegenerative disease, and/or evaluating progression of the neurodegenerative disease. The system comprises a substance for detecting a DNA damage-inducible transcript 4-like (DDIT4L) intron retention spliced product (DIR), and/or a nucleic acid encoding the DIR.

In some embodiments, the DIR comprises the amino acid sequence as set forth in SEQ ID NO: 1.

In some embodiments, the nucleic acid encoding the DIR comprises a nucleotide sequence as set forth in SEQ ID NO: 8.

In some embodiments, the nucleic acid encoding the DIR comprises a nucleotide sequence as set forth in SEQ ID NO: 93.

In some embodiments, the cognitive impairment comprises cognitive impairment caused by normal aging, Lewy body dementia (LBD), frontotemporal dementia and/or vascular dementia.

In some embodiments, the cognitive impairment-inducing diseases comprise Alzheimer's disease, multi-infarct type, Parkinson's disease, AIDS and/or Creutzfeldt-Jakob disease (CJD).

In some embodiments, the cognitive impairment comprises mild cognitive impairment (MCI), intermediate cognitive impairment and late cognitive impairment.

In some embodiments, the cognitive impairment comprises multi-domain amnestic MCI (aMCI-m).

In some embodiments, the neurodegenerative disease comprises an acute neurodegenerative disease and a chronic neurodegenerative disease.

In some embodiments, the neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

In some embodiments, the neurodegenerative disease comprises Alzheimer's disease, Parkinson's disease, multiple sclerosis (MS), amyotrophic lateral sclerosis (ALS) and/or Huntington's disease (HD).

In some embodiments, the neurodegenerative disease comprises presenile dementia.

In some embodiments, the neurodegenerative disease comprises early presenile dementia, intermediate presenile dementia and/or late presenile dementia.

In some embodiments, the system exists in a kit form.

In some embodiments, the system comprises a collection module capable of collecting a sample from a subject in need thereof.

In some embodiments, the subject comprises a mammal.

In some embodiments, the subject comprises a tumor patient.

In some embodiments, the subject is in an old age stage.

In some embodiments, the sample comprises a blood sample and/or a tissue or cell sample.

In some embodiments, the sample comprises whole blood, serum, plasma, and/or cerebrospinal fluid.

In some embodiments, the system comprises a detection module capable of detecting a content value of the DIR and/or of the nucleic acid encoding the DIR.

In some embodiments, the detection module detects a content value of the DIR and/or of the nucleic acid encoding the DIR in the sample acquired by the collection module.

In some embodiments, the detection module comprises a substance capable of detecting a content value of the DIR and/or of the nucleic acid encoding the DIR.

In some embodiments, the substance comprises an antigen-binding protein capable of specifically binding to the DIR, a probe capable of specifically binding to the DIR, a primer capable of specifically amplifying the nucleic acid encoding the DIR, a gene chip capable of specifically detecting the nucleic acid encoding the DIR, an aptamer capable of specifically binding to the nucleic acid encoding the DIR, and/or a guide RNA capable of specifically binding to the nucleic acid encoding the DIR.

In some embodiments, the antigen-binding protein comprises LCDR2, which comprises the amino acid sequence as set forth in SEQ ID NO: 74.

In some embodiments, the LCDR2 comprises the amino acid sequence as set forth in SEQ ID NO: 26 or 16.

In some embodiments, the antigen-binding protein comprises LCDR1, which comprises the amino acid sequence as set forth in SEQ ID NO: 73.

In some embodiments, the LCDR1 comprises the amino acid sequence as set forth in any one of SEQ ID NOs: 25, 52 and 15.

In some embodiments, the antigen-binding protein comprises LCDR3, which comprises the amino acid sequence as set forth in SEQ ID NO: 75.

In some embodiments, the LCDR3 comprises the amino acid sequence as set forth in any one of SEQ ID NOs: 27, 53, 67 and 17.

In some embodiments, the antigen-binding protein comprises LCDR1, LCDR2, and LCDR3, wherein
a) the LCDR1 comprises the amino acid sequence as set forth in SEQ ID NO: 25, the LCDR2 comprises the amino acid sequence as set forth in SEQ ID NO: 26, and the LCDR3 comprises the amino acid sequence as set forth in SEQ ID NO: 27;
b) the LCDR1 comprises the amino acid sequence as set forth in SEQ ID NO: 52, the LCDR2 comprises the amino acid sequence as set forth in SEQ ID NO: 26, and the LCDR3 comprises the amino acid sequence as set forth in SEQ ID NO: 53;
c) the LCDR1 comprises the amino acid sequence as set forth in SEQ ID NO: 52, the LCDR2 comprises the amino acid sequence as set forth in SEQ ID NO: 26, and the LCDR3 comprises the amino acid sequence as set forth in SEQ ID NO: 67; or,
d) the LCDR1 comprises the amino acid sequence as set forth in SEQ ID NO: 15, the LCDR2 comprises the amino acid sequence as set forth in SEQ ID NO: 16, and the LCDR3 comprises the amino acid sequence as set forth in SEQ ID NO: 17.

In some embodiments, the antigen-binding protein comprises HCDR1, which comprises the amino acid sequence as set forth in SEQ ID NO: 70.

In some embodiments, the HCDR1 comprises the amino acid sequence as set forth in any one of SEQ ID NOs: 20, 35, 56 and 10.

In some embodiments, the antigen-binding protein comprises HCDR2, which comprises the amino acid sequence as set forth in SEQ ID NO: 71.

In some embodiments, the HCDR2 comprises the amino acid sequence as set forth in any one of SEQ ID NOs: 21, 36, 42, 48 and 11.

In some embodiments, the antigen-binding protein comprises HCDR3, which comprises the amino acid sequence as set forth in SEQ ID NO: 70.

In some embodiments, the HCDR3 comprises the amino acid sequence as set forth in any one of SEQ ID NOs: 22, 30, 37, 43, 49, 57, 62 and 12.

In some embodiments, the antigen-binding protein comprises HCDR1, HCDR2, and HCDR3, wherein
a) the HCDR1 comprises the amino acid sequence as set forth in SEQ ID NO: 20, the HCDR2 comprises the amino acid sequence as set forth in SEQ ID NO: 21, and the HCDR3 comprises the amino acid sequence as set forth in SEQ ID NO: 22;
b) the HCDR1 comprises the amino acid sequence as set forth in SEQ ID NO: 20, the HCDR2 comprises the amino acid sequence as set forth in SEQ ID NO: 21, and the HCDR3 comprises the amino acid sequence as set forth in SEQ ID NO: 30;
c) the HCDR1 comprises the amino acid sequence as set forth in SEQ ID NO: 35, the HCDR2 comprises the amino acid sequence as set forth in SEQ ID NO: 36, and the HCDR3 comprises the amino acid sequence as set forth in SEQ ID NO: 37;
d) the HCDR1 comprises the amino acid sequence as set forth in SEQ ID NO: 20, the HCDR2 comprises the amino acid sequence as set forth in SEQ ID NO: 42, and the HCDR3 comprises the amino acid sequence as set forth in SEQ ID NO: 43;
e) the HCDR1 comprises the amino acid sequence as set forth in SEQ ID NO: 20, the HCDR2 comprises the amino acid sequence as set forth in SEQ ID NO: 48, and the HCDR3 comprises the amino acid sequence as set forth in SEQ ID NO: 49;
f) the HCDR1 comprises the amino acid sequence as set forth in SEQ ID NO: 56, the HCDR2 comprises the amino acid sequence as set forth in SEQ ID NO: 36, and the HCDR3 comprises the amino acid sequence as set forth in SEQ ID NO: 57;
g) the HCDR1 comprises the amino acid sequence as set forth in SEQ ID NO: 35, the HCDR2 comprises the amino acid sequence as set forth in SEQ ID NO: 36, and the HCDR3 comprises the amino acid sequence as set forth in SEQ ID NO: 62;
h) the HCDR1 comprises the amino acid sequence as set forth in SEQ ID NO: 35, the HCDR2 comprises the amino acid sequence as set forth in SEQ ID NO: 42, and the HCDR3 comprises the amino acid sequence as set forth in SEQ ID NO: 37;
i) the HCDR1 comprises the amino acid sequence as set forth in SEQ ID NO: 56, the HCDR2 comprises the amino acid sequence as set forth in SEQ ID NO: 36, and the HCDR3 comprises the amino acid sequence as set forth in SEQ ID NO: 57; or
j) the HCDR1 comprises the amino acid sequence as set forth in SEQ ID NO: 10, the HCDR2 comprises the amino acid sequence as set forth in SEQ ID NO: 11, and the HCDR3 comprises the amino acid sequence as set forth in SEQ ID NO: 12.

In some embodiments, the antigen-binding protein comprises a heavy chain variable region VH, which comprises the amino acid sequence as set forth in any one of SEQ ID NOs: 13, 23, 31, 38, 44, 50, 58, 63 and 65.

In some embodiments, the antigen-binding protein comprises a light chain variable region VL, which comprises the amino acid sequence as set forth in any one of SEQ ID NOs: 18, 28, 33, 40, 46, 54, 60 and 68.

In some embodiments, the antigen-binding protein comprises a heavy chain variable region VH and a light chain variable region VL, wherein:
a) the VH comprises the amino acid sequence as set forth in SEQ ID NO: 23 and the VL comprises the amino acid sequence as set forth in SEQ ID NO: 28;
b) the VH comprises the amino acid sequence as set forth in SEQ ID NO: 31 and the VL comprises the amino acid sequence as set forth in SEQ ID NO: 33;
c) the VH comprises the amino acid sequence as set forth in SEQ ID NO: 38 and the VL comprises the amino acid sequence as set forth in SEQ ID NO: 40;
d) the VH comprises the amino acid sequence as set forth in SEQ ID NO: 44 and the VL comprises the amino acid sequence as set forth in SEQ ID NO: 46;
e) the VH comprises the amino acid sequence as set forth in SEQ ID NO: 50 and the VL comprises the amino acid sequence as set forth in SEQ ID NO: 54;
f) the VH comprises the amino acid sequence as set forth in SEQ ID NO: 58 and the VL comprises the amino acid sequence as set forth in SEQ ID NO: 60;
g) the VH comprises the amino acid sequence as set forth in SEQ ID NO: 63 and the VL comprises the amino acid sequence as set forth in SEQ ID NO: 60;
h) the VH comprises the amino acid sequence as set forth in SEQ ID NO: 65 and the VL comprises the amino acid sequence as set forth in SEQ ID NO: 54;
i) the VH comprises the amino acid sequence as set forth in SEQ ID NO: 58 and the VL comprises the amino acid sequence as set forth in SEQ ID NO: 68; or
j) the VH comprises the amino acid sequence as set forth in SEQ ID NO: 13, and the VL comprises the amino acid sequence as set forth in SEQ ID NO: 18.

In some embodiments, the antigen-binding protein comprises a heavy chain constant region, which comprises an IgG-derived constant region.

In some embodiments, the heavy chain constant region comprises a constant region derived from a protein selected from the group consisting of: IgG1, IgG2, IgG3 and IgG4.

In some embodiments, the antigen-binding protein comprises a light chain constant region, which comprises an Igκ-derived constant region or an Igλ-derived constant region.

In some embodiments, the light chain constant region comprises a human Igκ-derived constant region.

In some embodiments, the antigen-binding protein comprises an antibody or an antigen-binding fragment thereof.

In some embodiments, the antigen-binding fragment is selected from the group consisting of: Fab, Fab', F(ab)2, an Fv fragment, F(ab')2, scFv, di-scFv, VHH and/or dAb.

In some embodiments, the antibody is selected from the group consisting of: a monoclonal antibody, a murine antibody and a chimeric antibody.

In some embodiments, the detection module comprises an instrument capable of detecting a content of the DNA damage-inducible transcript 4-like (DDIT4L) intron retention spliced product (DIR), and/or of the nucleic acid encoding the DIR.

In some embodiments, the detection module comprises a substance and/or an instrument capable of detecting a content of a marker associated with the cognitive impairment and/or the neurodegenerative disease.

In some embodiments, the marker associated with the cognitive impairment and/or the neurodegenerative disease comprises AD7C-NTP, pTau-181, pTau-217 and /or Aβ1-42.

In some embodiments, the detection module outputs a content value of the DIR and/or of the nucleic acid encoding the DIR.

In some embodiments, the system comprises a determination module capable of determining a diagnosis result and/or a disease progression result of said subject based on the content of said DIR and/or of said nucleic acid encoding said DIR as well as said normal control value and/or said early control value; the normal control value comprises a content of the DIR and/or of the nucleic acid encoding the DIR in a normal subject; and wherein the early control value comprises a previously measured content of the DIR and/or of the nucleic acid encoding the DIR in the same subject.

In some embodiments, the normal subject does not suffer from the cognitive impairment and/or the neurodegenerative disease.

In some embodiments, when the content of the DIR and/or of the nucleic acid encoding the DIR in the sample of the subject is significantly higher than the normal control value, the determination module diagnoses the subject with the cognitive impairment and/or the neurodegenerative disease.

In some embodiments, the subject has been diagnosed with the cognitive impairment and/or the neurodegenerative disease.

In some embodiments, when the content of the DIR and/or of the nucleic acid encoding the DIR in the sample of the subject is significantly higher than the early control value, the determination module diagnoses the subject with aggravated progression of the cognitive impairment and/or of the neurodegenerative disease.

In some embodiments, the determination module outputs a diagnosis result of the subject.

In some embodiments, the system comprises a display module capable of displaying the diagnosis result of the determination module.

In some embodiments, the system comprises a storage module capable of storing the content value output by the detection module, and/or the diagnosis result output by the determination module.

In another aspect, the present application provides a medicament screening method, comprising the step of: detecting changes in a content of DIR and/or of a nucleic acid encoding the DIR in a subject after administration of a candidate medicament, wherein the medicament is for use in prevention, treatment and/or alleviation of cognitive impairment and/or a neurodegenerative disease.

In some embodiments, the DIR comprises the amino acid sequence as set forth in SEQ ID NO: 1.

In some embodiments, the nucleic acid encoding the DIR comprises a nucleotide sequence as set forth in SEQ ID NO: 8.

In some embodiments, the nucleic acid encoding the DIR comprises a nucleotide sequence as set forth in SEQ ID NO: 93.

In some embodiments, the cognitive impairment comprises cognitive impairment caused by normal aging, Lewy body dementia (LBD), frontotemporal dementia and/or vascular dementia. In some embodiments, the cognitive impairment-inducing diseases comprise Alzheimer's disease, multi-infarct type, Parkinson's disease, AIDS and/or Creutzfeldt-Jakob disease (CJD).

In some embodiments, the cognitive impairment comprises mild cognitive impairment (MCI), intermediate cognitive impairment and late cognitive impairment.

In some embodiments, the cognitive impairment comprises multi-domain amnestic MCI (aMCI-m).

In some embodiments, the neurodegenerative disease comprises an acute neurodegenerative disease and a chronic neurodegenerative disease.

In some embodiments, the neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

In some embodiments, the neurodegenerative disease comprises Alzheimer's disease, Parkinson's disease, multiple sclerosis (MS), amyotrophic lateral sclerosis (ALS) and/or Huntington's disease (HD).

In some embodiments, the neurodegenerative disease comprises presenile dementia.

In some embodiments, the neurodegenerative disease comprises early presenile dementia, intermediate presenile dementia and/or late presenile dementia.

In some embodiments, when the content of the DIR and/or of the nucleic acid encoding the DIR in the subject is reduced due to administration of the candidate medicament, the candidate medicament has a therapeutic effect.

In some embodiments, the administration comprises injection.

In some embodiments, the medicament comprises a small molecule medicament and/or a bio-macromolecular medicament.

In the first aspect of the present application, there is provided application of a substance for detecting an intron retention spliced product (DIR) from a sample of a subject for encoding a DNA damage-inducible transcript 4-like (DDIT4L) in preparation of a product (e.g., a kit) for diagnosis of presenile dementia and mild cognitive impairment and/or evaluation of progression (e.g., grading or staging) of the presenile dementia in the subject.

In some embodiments, the subject is selected from: humans, non-human primates, rodents (such as rats, mice, and guinea pigs), dogs, cats, horses, cattle, and sheep.

In some embodiments, the subject does not suffer from cancer.

In some embodiments, the subject does not suffer from a malignant tumor.

In some embodiments, the DIR has a sequence selected from the group consisting of: a nucleotide sequence (mRNA) of SEQ ID NO: 2; an encoding nucleotide molecule of a nucleotide sequence as set forth in SEQ ID NO: 3, corresponding to a DIR peptide; or a DIR peptide having the amino acid sequence as set forth in SEQ ID NO: 4; a variant derived from the above sequences by substitution, deletion and/or addition of one or more nucleotides and/or amino acids; and a sequence having a high degree of homology (e.g., higher than 80%, 85%, 90%, 95%, 96%, 97%, 98%, 99%, 99.5% sequence homology) to the above sequences.

In some embodiments, the presenile dementia comprises an early stage (an amnesia stage), an intermediate stage (a neurological disorder stage) and a late stage (a severe dementia stage).

In some embodiments, the substance is selected from a reagent, an apparatus, or their combination for detecting the DIR.

In some embodiments, the substance is the one used to detect the DIR at the gene level and/or at the protein level. For example, the substance is the one used in one or more detection techniques or methods selected from the group consisting of: immunohistochemistry (such as immunofluorescence analysis, chemiluminescence, reverse enzyme-linked immunosorbent assay, and an immunocolloidal gold method), Western blotting, Northern blotting, PCR, a biochip method, and a protein chip method.

In some embodiments, the substance is selected from: a substance specific to the DIR, for example, an antibody (e.g., a monoclonal antibody) of the DIR; a probe, gene chip, PCR primer, or gRNA specific to the spliced product, etc.

In some embodiments, the sample is selected from: a blood sample, such as whole blood, serum, and plasma; a tissue or cell sample; and cerebrospinal fluid.

In some embodiments, the blood sample is fresh blood or cryopreserved blood.

In some embodiments, an increase in the DIR level in the sample compared to a normal control value indicates that the subject suffers from presenile dementia or mild cognitive impairment.

In some embodiments, an increase in the DIR level in the sample compared to a mild cognitive impairment control or an early control level in a subject who has been diagnosed with presenile dementia indicates some development (e.g., a higher grade or stage) of the presenile dementia in the subject.

In some embodiments, an increase in the DIR level in the sample compared to a low-grade or low-stage control value indicates some development (e.g., a higher grade or stage) of the presenile dementia in the subject.

In another aspect, the present application provides a diagnostic kit, comprising a substance as described in the present application capable of detecting a DNA damage-inducible transcript 4-like (DDIT4L) intron retention spliced product (DIR), and/or a nucleic acid encoding the DIR.

For example, the present application provides a product (e.g., kit) for diagnosis of presenile dementia and mild cognitive impairment, and/or for evaluation of progression (e.g., a grade or stage) of the presenile dementia, comprising:
(i) a substance for detecting DIR in a sample from a subject; and
(ii) optionally, other substances for diagnosis of the presenile dementia and mild cognitive impairment, and/or for evaluation of progression (e.g., a grade or stage) of the presenile dementia, for example, a substance for detecting an existing marker for the presenile dementia.

In some embodiments, the substance in (i) is selected from one or more of the group consisting of:
a reagent, an apparatus or their combination for detecting the DIR;
a substance for detecting the DIR at the gene level and/or protein level, such as a substance for use in one or more detection techniques or methods selected from the group consisting of: immunohistochemistry (e.g., immunofluorescence analysis, chemiluminescence, reverse enzyme-linked immunosorbent assay, an immunocolloidal gold method), Western blotting, Northern blotting, PCR, and a biochip method; or
a substance specific to the DIR, for example, an antibody (preferably, a monoclonal antibody) of the substance; a probe, gene chip, PCR primer, or gRNA specific to the spliced product.

In some embodiments, other substances in (ii) are selected from: a substance for detecting one or more other markers for presenile dementia, for example: AD7C-NTP, pTau-181, pTau-217, and Aβ1-42; and/or, a reagent for imaging of brain amyloid protein; and/or, a reagent for diagnosis of the imaging of the subject's brain.

In some aspects of the present application, there is provided a method for screening of a medicament for treatment of presenile dementia. The method comprises: detecting an effect of a candidate medicament on the DIR level in a subject, wherein after the use of the candidate medicament, the decreased level indicates that the candidate medicament has a therapeutic effect on the presenile dementia.

In some embodiments, the detecting is performed using the product as described in the present application.

In some aspects of the present application, there is provided a method for diagnosis of presenile dementia and mild cognitive impairment, and/or for evaluation of progression of the cognitive impairment. The method comprises: detecting a DIR level in a sample from the subject, and accordingly performing diagnosis of presenile dementia and/or evaluation of progression (e.g., grading or staging) of the presenile dementia on the subject.

In some embodiments, the detecting is performed using the product as described in the present application.

In some embodiments, an increase in the DIR level in the sample compared with the control value indicates that the subject suffers from presenile dementia; or, an increase in the DIR level in the sample compared with a low-grade or low-stage control indicates some development (e.g., a higher grade or stage) of the presenile dementia in the subject.

In some aspects of the present application, there is also provided a system corresponding to the method of the present application. The system comprises the following devices:
(a) a device for collecting and/or receiving data on a DIR level in a sample; and
(b) a device for analyzing the data for diagnosis of presenile dementia and mild cognitive impairment and/or for evaluation of progression (e.g., grading or staging) of the presenile dementia in the subject, wherein an increase in the DIR level in the sample compared to the normal control value indicates that the subject suffers from the presenile dementia or mild cognitive impairment; or, an increase in the DIR level in the sample compared to a mild cognitive impairment control or an early control level in a subject who has been diagnosed with presenile dementia indicates some development (e.g., a higher grade or stage) of the presenile dementia in the subject.

In some embodiments, the system further comprises (a') a device for collecting and/or receiving presenile dementia diagnosis and/or data on other clinical indicators or basic characteristics of the development of the presenile dementia in a sample.

In some embodiments, the device described in (b) is used to combine and analyze DIR level data with data on other clinical indicators or basic characteristics of presenile dementia diagnosis and/or development progression of presenile dementia, in order to perform prognosis evaluation on the presenile dementia diagnosis and/or the development progression of presenile dementia of the subject.

Or, in some embodiments, the system further comprises a device for analyzing presenile dementia diagnosis and/or data on other clinical indicators or basic characteristics of the development progression of presenile dementia; and/or a device for combining and analyzing DIR level data with the data on other clinical indicators or basic characteristics of presenile dementia diagnosis and/or the development progression of presenile dementia, in order to evaluate the presenile dementia diagnosis and/or the development progression of presenile dementia in the subject.

In some embodiments, the system further comprises one or more devices selected from the group consisting of: a device for inputting and/or outputting and/or storing data; a device for outputting and/or storing analysis results, for example, a device for uploading and/or storing the analysis results to a cloud database or its corresponding cloud database device; and a device for performing intelligent analysis and recommending a therapeutic scheme based on prognosis evaluation results.

It should be understood that those skilled in the art may add other conventional devices or programs to the system as needed.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the invention involved in the present application are listed in the appended claims. The characteristics and advantages of the invention involved in the present application may be better understood by referring to the exemplary embodiments and the accompanying drawings described in detail below. A brief description of the drawings is as follows:

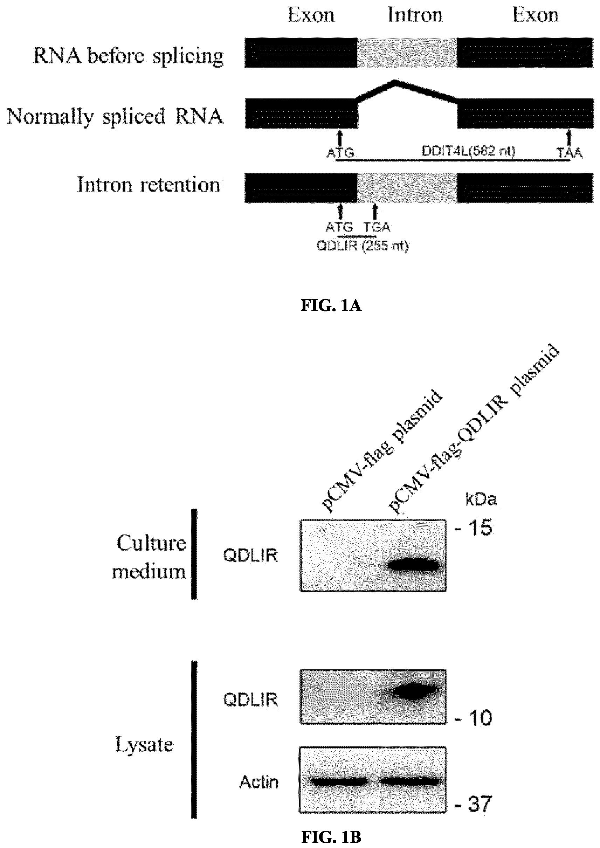

FIGs. 1A to 1B show the splicing of a DDIT4L mRNA precursor and the secretion of a DIR protein from transfected cells into a medium. In the figures: FIG. 1A shows the splicing of DDIT4L, in which the length of the mRNA obtained by normal shearing of an intron is 582 nt (SEQ ID NO: 95) and the length of an RNA encoding the DIR in the mRNA obtained by abnormal splicing of intron retention is 255 nt (SEQ ID NO: 8); and FIG. 1b shows the secretion of the DIR from cells into a medium, in which a pCMV-flag-DIR plasmid is transfected into HEK293 cells and cultured for 48 h, followed by the observation that a secreted DIR protein is detectable in the medium. DIR protein bands are visible in DIR plasmid lane of the lysate, which are 12 kDa in size.
FIGs. 2A to 2B show changes in the content of DIR protein in the blood of AD patients and of patients with a variety of other diseases. FIG. 2A shows changes in the content of DIR in the blood of AD patients and of patients with a variety of other diseases by Western assay; and the plasma protein in the figure serves as the light chain of immunoglobulins that act as an internal reference. FIG. 2A shows changes in the content of DIR in the blood of AD patients, MCI patients, and non-AD/MCI patients by ELISA assay. In the figures, *** represents P < 0.001, and * represents P < 0.05.
FIG. 3 shows a standard curve of a standard DIR protein detected by ELISA.
FIG. 4 shows a R°C curve of a clinical sample detected by ELISA.
FIG. 5 shows changes in the content of DIR in the blood of patients with different classes of MCI.
FIG. 6 shows changes in the content of DIR and other indicators in the blood of patients with different classes of MCI and AD.
FIG. 7 shows the correlation of DIR expression levels in plasma and cerebrospinal fluid.
FIG. 8 shows the detection results on the binding activity of the DIR antibody described in the present application.

### DETAILED DESCRIPTION OF THE PREFERRED EMBODIMENTS

The embodiments of the invention of the present application will be illustrated by specific examples below. Those familiar with this technology may easily understand other advantages and effects of the invention of the present application from the content disclosed in the specification.

### TERMS & DEFINITIONS

The term "DDIT4L" generally refers to DNA damage-inducible transcript 4-like. It can also be called REDD2/RTP801L. Studies have found that DDIT4L can be associated with cardiac dysfunction. It can also be used to treat gliomas. The accession number of a human DDIT4L gene in GenBank is 115265; the accession number of a human DDIT4L protein in GenBank is NP_660287.1.

The term "QDLIR" generally refers to the intron retention spliced product of DDIT4L. In the present application, the amino acid sequence of the QDLIR may be as set forth in SEQ ID NO: 1. In the present application, QDLIR can be used interchangeably with DIR, and it can be an intron retention form produced by abnormal splicing during expression of the human DDIT4L gene. In the present application, a gene encoding the DIR may be referred to as a DIR gene. DIR may comprise the amino acid sequence encoded by a first exon (with the amino acid sequence as set forth in SEQ ID NO. 2) and the amino acid sequence encoded by a retained intron (with the amino acid sequence as set forth in SEQ ID NO. 3). Here, the amino acid sequence (IR) encoded by the retained intron may be divided into two parts, namely, IR-I (i.e. DIR-I, with the amino acid sequence as set forth in SEQ ID NO. 4) consisting of the first 27 amino acids from the N-terminus, and DIR-II (i.e., DIR-II, with the amino acid sequence as set forth in SEQ ID NO. 5) consisting of the last 27 amino acids. The term "expression level" generally refers to a protein, RNA or mRNA level of a specific related gene. Any method known in the art can be used to determine the expression level of a specific related gene (e.g., a human DDIT4L gene). In the present application, the "expression" generally refers to the process of transforming the information encoded by a gene into a structure that exists in and operates in a cell. For example, reverse transcription and amplification analysis (e.g., PCR, ligation-dependent RT-PCR or quantitative RT-PCT), hybridization analysis, Northern blotting, dot blotting, in situ hybridization, gel electrophoresis, capillary electrophoresis, column chromatography, Western blotting, immunohistochemistry, immunostaining, or mass spectrometry can be included. A biological sample or a protein/nucleic acid isolated from the sample can be analyzed directly.

The term "neurodegenerative disease" generally refers to dementia or other cognitive impairment resulting from the gradual loss of neuronal structures and functions, including neuronal death and glial cell homeostasis. In some cases, age (e.g., Alzheimer's disease (AD), Parkinson's disease (PD)) or genetic mutations (e.g., Huntington's disease, early-onset AD or PD, amyotrophic Lateral sclerosis (ALS)) that affect CNS cell function may induce the neurodegenerative disease. The neurodegenerative disease may have changes and/or disorders selected from the group consisting of: protein misfolding and aggregation; neuroinflammation (e.g., CNS inflammation occurring in response to signal stimuli such as toxic stimuli (e.g., protein aggregation), infection, traumatic injury, or autoimmune); changes in cell signaling; acquired senescence/cell death (e.g., disrupted apoptotic signaling, mitochondrial dysfunction, impaired autophagy, and activation of necrosomes by stress/inflammation); and motor cell damage and epigenetic changes.

The term "Alzheimer's disease" generally refers to presenile dementia or senile dementia, which is a neurodegenerative disease that develops slowly and gets worse over time. The most common early symptom is loss of short-term memory (having difficulty in remembering recent events). As the disease progresses, at least one of the following symptoms may gradually appear: language disorder, disorientation (for example, getting lost easily), emotional lability, loss of motivation, inability to take care of oneself and behavioral problems. The true cause of Alzheimer's disease remains unknown, but its progression may be related to the deposition of fibrillar amyloid protein plaques and tau protein in the brain. At present, there is no therapy that can stop or reverse the course of the disease, but a few methods that may temporarily relieve or ameliorate symptoms.

The term "presenile dementia" is used interchangeably with the term "Alzheimer's disease" in the present application. The presenile dementia comprises early presenile dementia, intermediate presenile dementia and/or late presenile dementia. For example, the learning and memory impairment of the patients with early presenile dementia will become more and more obvious. In some cases, they will develop language disorder, executive impairment, cognitive impairment (agnosia) and/or skill execution impairment (apraxia). For example, the patients with intermediate presenile dementia will lose the ability to live independently and may be unable to carry out most of daily activities (in some cases, they may suffer from anomia, paraphasia, and/or anosognosia). For example, the patients with late presenile dementia may depend on caregivers in the late stage. For example, they may completely lose language competence. For example, they may not be able to feed themselves.

The term "cognitive impairment" generally refers to a disease and condition that is considered to or do involve the progressive loss of neuronal structure and/or function (including neuronal death) or those described above. For example, the characteristics of cognitive impairment may comprise impairment of cognition (e.g., memory, attention, perception, and/or thinking). These impairment may comprise pathogen-induced cognitive dysfunction, for example, HIV-related cognitive dysfunction and Lyme disease-related cognitive dysfunction. Examples of cognitive impairment may comprise Alzheimer's disease, Huntington's disease, Parkinson's disease, amyotrophic lateral sclerosis (ALS), autism, mild cognitive impairment (MCI), stroke, traumatic brain injury (TBI) and/or age-associated memory impairment (AAMI).

The term "mild cognitive impairment (MCI)" generally refers to an intermediate clinical state between normal cognition and cognitive impairment. In some cases, the MCI may comprise cognitive impairment that meets criteria for dementia but is worse than normal aging. MCI is diverse in clinical manifestation, etiology, prognosis, and prevalence. In some cases, MCI may be a pathological stage of Alzheimer's disease. Some forms of cognitive impairment may be considered as early manifestations of the neurodegenerative disease and may eventually lead to dementia. In some cases, the MCI may comprise a subtype selected from the group consisting of: aMCI-s: single-domain amnestic MCI; aMCI-m: multi-domain amnestic MCI; naMCI-s: single-domain non-amnestic MCI; and naMCI-m: multi-domain non-amnestic MCI.

The term "cognitive impairment caused by normal aging" generally refers to cognitive impairment due to normal aging. For example, the cognitive impairment caused by normal aging may be manifested as: memory loss, confusion about the location of familiar places, taking longer time than usual to complete daily tasks, or changes in mood and personality.

The term "Lewy body dementia (LBD)" generally refers to dementia with Lewy bodies. Dementia with Lewy bodies is characterized by abnormal accumulation of proteins into lumps called Lewy bodies. Dementia with Lewy bodies cause a gradual decline in mental ability. People with Lewy body dementia may experience visual hallucinations and changes in alertness and attention. Other influences comprise muscle stiffness, slowness of movement, difficulty in walking and tremors. Patients with Lewy bodies in the brain may also have plaques and tangles associated with Alzheimer's disease.

The term "frontotemporal dementia" generally refers to Pick's disease, which is a rare, progressive disease in which a tau protein affects only the frontal and temporal lobes of the brain. Patients with frontotemporal dementia have difficulty in higher-level reasoning, language expression, speech perception, and memory formation. In patients with frontotemporal dementia, the frontal and temporal lobes of the brain may atrophy over time.

The term "vascular dementia" generally refers to problems with reasoning, judgment, and memory caused by impaired blood flow to the brain. For example, the vascular dementia may comprise dementia caused by risk factors for heart disease and stroke, such as high blood pressure and high cholesterol.

The term "multi-infarct type" generally refers to small noncortical infarcts caused by occlusion of a single perforating branch of a large cerebral artery. The multi-infarct type may be a special type of cerebral infarction, also known as ischemic stroke. The multi-infarct type may be manifested as hemidysesthesia, aphasia, anarthria, slow movement, and clumsiness (especially difficulty with fine movements such as writing).

The term "Parkinson's disease" generally refers to a progressive neurodegenerative disease. The clinical features of Parkinson's disease (PD) may comprise motor symptoms (e.g., tremor, bradykinesia, myotonia, and postural instability), as well as neuropsychiatric and other non-motor manifestations. For example, the non-motor manifestations may comprise cognitive dysfunction and dementia, mood disorders (e.g., depression, anxiety, apathy), and sleep disorders.

The term "Creutzfeldt-Jakob disease (CJD)" generally refers to a type of transmissible spongiform encephalopathy that occurs in humans. CJD is a disease caused by prion infection. CJD patients may manifest paranoid behavior, confusion, loss of appetite and weight, and depression, and a few patients have visual or auditory abnormalities; and in the progressive stage, they manifest progressive neurological deterioration (e.g., sensory abnormalities, language disorder, and aphasia).

The term "multiple sclerosis (MS)" generally refers to a demyelinating neuropathy. Insulating substances (i.e., myelin sheath) on the surfaces of nerve cells in the brain or spinal cord of the MS patients are damaged, and the signal transduction of the nervous system is damaged, which can lead to a series of possible symptoms, affecting the activity, mental ability, and even mental state. These symptoms may comprise diplopia, unilateral visual impairment, myasthenia, dysesthesia, or dystaxia.

The term "amyotrophic lateral sclerosis (ALS)" generally refers to a motor neuron disease, which is a progressive and fatal neurodegenerative disease. A small number of ALS patients may develop frontotemporal lobe dementia. Some ALS patients may experience deterioration in their senses of sight, touch, smell, and taste, and a very small number of ALS patients may develop dementia at the same time.

The term "Huntington's disease (HD)" generally refers to a genetic disease that causes brain cell death. With the progress of this disease, HD patients may experience more obvious incoordination of body movements, and their ability gradually deteriorates until movement becomes difficult and they cannot speak. Then, the mental ability generally declines into dementia.

The term "aging stage" generally refers to a subject's stage of aging. For example, for humans, the aging stage may be over 60 years old, over 70 years old, or over 75 years old; and for mice, the aging stage may be over 10 months old, for example, over 13 months old or over 18 months old. In some cases, a subject in the aging stage may have one or more symptoms of learning deficit, memory impairment, memory deficit, and/or brain dysfunction.

The term "variant" generally refers to a polypeptide comprising the amino acid sequence that differs by at least one amino acid residue from the amino acid sequence of a parent or reference polypeptide (e.g., a wild-type polypeptide). In the present application, the variant may have a high (e.g., at least 80%) homology to the parent or reference polypeptide. The homology may comprise sequence similarity or identity. In the present application, the homology may be determined using standard techniques known in the art (see, for example, Smith and Waterman, "Advances in Applied Mathematics"); and the percentage of identity shared by polynucleotide or polypeptide sequences is determined by direct comparison of sequence information between molecules, and the comparison is performed by sequence alignment to determine the identity using methods known in the art. An example of an algorithm suitable for determining sequence similarity is the BLAST algorithm (see Altschul et al., Journal of Molecular Biology, 215: 403-410 [1990]). Software for BLAST analyzes is publicly available through the National Center for Biotechnology Information (NCBI).

In the present application, the term "CDR", short for "complementary determining region", generally refers to a region of an antibody variable domain, and its sequence is highly variable and/or forms a structure-defined ring. For example, the antibody may comprise six CDRs, with three (HCDR1, HCDR2, and HCDR3) in VH and three (LCDR1, LCDR2, and LCDR3) in VL. In some embodiments, a naturally occurring camelid antibody composed of heavy chains only still functions normally and stably in the absence of light chains. The CDR of antibody can be determined by a variety of coding systems, such as CCG, Kabat, Chothia, IMGT, the combination of Kabat/Chothia, etc. These encoding systems are known in the art. See, for example, www.bioinf.org.uk/abs/index.html#kabatnum for details. For example, the amino acid sequence number of the antigen-binding protein can be numbered according to the IMGT numbering scheme (IMGT, the international ImMunoGeneTics information system@imgt.cines.fr; imgt.cines.fr; Lefranc et al., 1999, Nucleic Acids Res. 27: 209-212; Ruiz et al., 2000 Nucleic Acids Res. 28: 219-221; Lefranc et al., 2001, Nucleic Acids Res. 29: 207-209; Lefranc et al., 2003, Nucleic Acids Res. 31: 307-310; Lefranc et al., 2005, DevComp Immunol 29: 185-203). For example, the CDR of the antigen-binding protein can be determined according to the Kabat numbering system (see, for example, Kabat EA &Wu TT (1971) Ann NY Acad Sci 190:382-391 and Kabat EA et al., (1991) Sequences of Proteins of Immunological Interest, Fifth Edition, U.S. Department of Health and Human Services, NIH Publication No.91-3242).

In the present application, the term "FR" generally refers to a more highly conserved part in the variable domain of an antibody, and it is referred to as a framework region. For example, the variable domains of naturally occurring heavy and light chains may each comprise four FRs, namely four (H-FR1, H-FR2, H-FR3, and H-FR4) in VH, and four (L-FR1, L-FR2, L-FR3, and L-FR4) in VL.

In the present application, the terms "variable domain" and "variable region" are used interchangeably and each generally refer to a portion of an antibody heavy chain and/or light chain. The variable domains of the heavy and light chains may be referred to as "VH" and "VL" respectively (or "VH" and "VL" respectively). These domains are generally the most varied fractions of an antibody (with respect to other antibodies of the same type) and may comprise antigen-binding sites. In the present application, the term "variable" generally refers to the fact that certain segments of the variable domains may differ greatly in sequence between antibodies. A variable domain-mediated antigen may bind and determine the specificity of a specific antibody to its specific antigen. However, the variability may not be evenly distributed across the entire range of variable domain. It may be generally concentrated in three segments called hypervariable regions (CDRs or HVRs) in the variable domains of the light and heavy chains. The more highly conserved part of the variable domain may be referred to as framework region (FR). The variable domains of the naturally occurring heavy and light chains may each comprise four FRs, most of which have a β-pleated configuration and which are linked by three CDRs to form a circular linkage, and to form a fraction of a β-pleated structure in some cases. The CDRs in each chain may be held together closely by FR regions, and the CDRs from another chain jointly promote the formation of the antigen-binding site of the antibody.

In the present application, the term "antibody" generally refers to an immunoglobulin or a fragment or derivative thereof, and encompasses any polypeptide comprising an antigen-binding site, regardless of its production in vitro or in vivo. The term may comprise, but is not limited to, polyclonal, monoclonal, monospecific, multispecific, non-specific, humanized, single-chain, chimeric, synthetic, recombinant, hybrid, mutant and grafted antibodies. Unless otherwise modified by the term "intact" (as in "intact antibody"), for the purposes of the present invention, the term "antibody" may also comprise antibody fragments, such as Fab, F(ab')2, Fv, scFv, Fd, VHH, dAb, and other antibody fragments that maintain the antigen-binding function (for example, specific binding to human DIR). Generally, such fragments may comprise antigen-binding domains. A basic 4-chain antibody unit may be a heterotetrameric glycoprotein composed of two identical light (L) chains and two identical heavy (H) chains. An IgM antibody may be composed of 5 basic heterotetrameric units and another polypeptide called J chain, and comprise 10 antigen-binding sites; and an IgA antibody may comprise 2-5 basic 4-chain units that may bind to and be polymerized with the J chain to form a multivalent combination. In the case of IgG, the 4-chain unit may be generally of about 150,000 Daltons. Each L chain may be linked to the H chain by a covalent disulfide bond, and two H chains may be linked to each other by one or more disulfide bonds depending on the isotype of the H chains. Each of the H and L chains may also have a regularly spaced intra-chain disulfide bridged bond. Each H chain may have a heavy chain variable domain (VH) at its N-terminus, followed by three constant domains (CH) for each of α and γ chains, or followed by four CH domains for the µ and ε isoforms. Each L chain may have a light chain variable domain (VL) at its N-terminus and a constant domain at the other end. VL may correspond to VH, and light chain constant region (CL) may correspond to a first constant domain (CH1) of the heavy chain. Specific amino acid residues may be considered to form an interface between the variable domains of the light and heavy chains. VH and VL may be paired together to form a single antigen-binding site. An L chain from any vertebrate species may be classified, based on the amino acid sequence of its constant domain, into one of two distinct types, called κ and λ. Immunoglobulins may be classified into different classes or isotypes depending on the amino acid sequences of constant domains of the heavy chains (CH). At present, there are five classes of immunoglobulins, namely, IgA, IgD, IgE, IgG (e.g., IgG1, IgG2, IgG3 and/or IgG4), and IgM, which have heavy chains named α, δ, ε, γ, and µ, respectively.

In the present application, the term "antigen-binding fragment" generally refers to one or more fragments capable of specifically binding to an antigen (for example, DIR). In the present application, the antigen-binding fragment may comprise Fab, Fab', F(ab)2, a Fv fragment, a F(ab')2, scFv, di-scFv, VHH and/or dAb.

In the present application, the term "Fab" generally refers to the antigen-binding fragment of an antibody. As described above, an intact antibody may be digested using papain. The antibody digested by the papain produces two identical antigen-binding fragments, namely, a "Fab" fragment, and a residual "Fc" fragment (i.e., an Fc region). The Fab fragment may be composed of a complete L chain, the variable region of a heavy chain and the first constant region (CH1) of the H chain (VH).

In the present application, the term "F(ab)2" generally refers to the antigen-binding fragment of an antibody. For example, F(ab)2 may be formed by linking two Fab fragments.

In the present application, the term "Fab'" generally refers to a monovalent antigen-binding fragment of a human monoclonal antibody, and the fragment is slightly larger than a Fab fragment. For example, a Fab' fragment may comprise all of the light chains, all of the variable regions of the heavy chain, and all or some of the first and second constant regions of the heavy chain. For example, the Fab' fragment may further comprise some or all of the 220-330 amino acid residues of the heavy chain.

In the present application, the term "F(ab')2" generally refers to an antibody fragment produced by pepsin digestion of an intact antibody. The F(ab')2 fragment comprises two Fab fragments held together by a disulfide bond, and some of hinge regions. The F(ab')2 fragment have a bivalent antigen-binding activity and is capable of cross-linking an antigen.

In the present application, the term "Fv fragment" generally refers to a monovalent antigen-binding fragment of a human monoclonal antibody, including all or some of the heavy-chain and light-chain variable regions, and lacking the heavy-chain and light-chain constant regions. The heavy-chain and light-chain variable regions comprise, for example, CDRs. For example, an Fv fragment comprises all or some of the amino-terminus variable regions of about 110 amino acids of the heavy and light chains.

In the present application, the term "scFv" generally refers to a fusion protein comprising at least one antibody fragment comprising a light chain variable region and at least one antibody fragment comprising a heavy chain variable region, wherein the light-chain and heavy-chain variable regions are contiguous (e.g., via a synthetic linker such as a short flexible polypeptide linker) and can be expressed as a single chain polypeptide, and wherein the scFv retains the specificity of the intact antibody from which it is derived. Unless otherwise stated, as used in the present application, the scFv may have the VL and VH variable regions described in any order (e.g., relative to the N-terminus and C-terminus of the polypeptide), and the scFv may comprise VL-linker-VH or VH-linker-VL.

In the present application, the term "dAb" generally refers to an antigen-binding fragment consisting of a VH domain or a VL domain. Refer to, for example, Ward et al. (Nature, 1989 Oct 12; 341(6242): 544-6), and Holt et al., Trends Biotechnol., 2003, 21(11): 484-490.

In the present application, the term "VHH" generally refers to an antibody comprising the antigen-binding variable domain of an antibody comprising a heavy chain (see Vanlandschoot P. et al., 2011, Antiviral Research, 92, 389-407). VHH may also be called nanobody (Nb).

In the present application, the term "monoclonal antibody" generally refers to an antibody molecule product consisting of single molecules. The monoclonal antibody is generally highly specific for a single antigen site. Moreover, unlike conventional polyclonal antibody preparations (which typically have different antibodies for different determinants), each monoclonal antibody can target a single determinant on an antigen. In addition to their specificity, the advantage of monoclonal antibodies lies in that they may be synthesized by hybridoma culture, without being contaminated by other immunoglobulins. The modifier "monoclonal" may indicate the antibody characteristics obtained from a substantially homogeneous population of antibodies, and should not interpreted as requiring the production of antibodies by any particular method. For example, the monoclonal antibodies used in the present application may be prepared in hybridoma cells, or may be prepared by recombinant DNA methods.

In the present application, the term "chimeric antibody" generally refers to an antibody in which the variable region is derived from one species and the constant region is derived from another species. Generally, the variable region is derived from an antibody ("parent antibody") in an experimental animal such as a rodent, and the constant region is derived from a human antibody, such that the possibility of causing an adverse immune response in an individual human by the resulting chimeric antibody is reduced as compared with the parental (for example, mouse-derived) antibody.

In the present application, the term "humanized antibody" generally refers to an antibody in which some or all of the amino acids outside the CDR of a non-human antibody (such as a mouse antibody) have been replaced by corresponding amino acids derived from human immunoglobulins. In the CDR, small additions, deletions, insertions, substitutions, or modifications to the amino acids may also be allowed, as long as they still retain the capability of the antibody to bind to a specific antigen. The humanized antibody may optionally comprise at least a portion of a constant region of a human immunoglobulin. A "humanized antibody" may retain the antigen specificity similar to that of the original antibody. The "humanized" form of a non-human (for example, mouse) antibody may minimally comprise a chimeric antibody derived from a non-human immunoglobulin sequence. In some cases, CDR residues in a human immunoglobulin (receptor antibody) may be replaced with CDR residues from a non-human species (donor antibody) (such as a mouse, a rat, a rabbit, or a non-human primate) with the desired properties, affinity, and/or capability. In some cases, FR residues of the human immunoglobulin may be replaced with corresponding non-human residues. In addition, the humanized antibody may comprise amino acid modifications that are not present in a receptor antibody or in a donor antibody. These modifications may be made to further improve the properties such as binding affinity of the antibody.

In the present application, the term "fully human antibody" generally refers to an antibody that comprises human immunoglobulin sequences only. A fully human antibody may comprise murine glycans if it is produced in mice, in mouse cells, or in hybridomas derived from mouse cells. Similarly, "murine antibody," "mouse antibody," or "rat antibody" refers to an antibody that comprises mouse or rat immunoglobulin sequences only, respectively. The fully human antibody may be produced in humans and in transgenic animals with human immunoglobulin germline sequences by phage display or other molecular biological methods. Exemplary techniques that can be used to manufacture antibodies are known in the art.

In the present application, the term "antigen-binding protein" generally refers to a protein comprising a moiety that binds to an antigen, and optionally a scaffold or skeleton moiety that allows the moiety that binds to the antigen to adopt a conformation that promotes the binding of the antigen-binding protein to the antigen. Examples of the antigen-binding protein comprise, but are not limited to, antibodies, antigen-binding fragments (Fab, Fab', F(ab)2, Fv fragments, F(ab')2, scFv, di-scFv, VHH and/or dAb), immune conjugates, multispecific antibodies (e.g., bispecific antibodies), antibody fragments, antibody derivatives, antibody analogs or fusion proteins, etc., as long as they demonstrate the desired antigen-binding activity. The "antigen-binding protein" of the present application may comprise an antigen-binding fraction and optionally, a scaffold or construct fraction allowing the antigen-binding fraction to adopt a conformation that facilitates the binding of the antigen-binding fraction to an antigen.

The term "about" generally refers to a numerical range that is 20% more or less than a specified value. For example, "about X" comprises a numeric range of X ± 20%, ±10%, ±5%, ±2%, ±1%, ±0.5%, ±0.2% or 0.1%, with X being a numerical value.

### DETAILED DESCRIPTION OF THE INVENTION

The prerequisite for early detection and early treatment of presenile dementia is to find its specific diagnostic markers. In the study, the inventor has found that the DIR protein is present in the blood of presenile dementia patients, but is not detected in the blood samples of normal humans. This result shows that DIR is likely to become a blood marker for the diagnosis (possibly early diagnosis) of presenile dementia. At the same time, the inventor also tested the blood samples from other patients with cognitive impairment and found the DIR protein level increased significantly in the blood samples from patients with MCI (mild cognitive impairment) and other cognitive impairments. Therefore, increased DIR in blood will be a new marker in the diagnosis and treatment of presenile dementia.

Specifically, under pathological conditions (with such as tumors, neurodegenerative diseases, etc.), the diseased tissue experiences hypoxia due to lack of blood supply, leading to a series of changes in gene expression, and at the same time, abnormal splicing forms appear. How to find the specific genes and their splicing forms may be the key to the treatment of diseases. In the previous studies, the inventor has found that DIR is an abnormal protein formed by abnormal splicing in a human body and only exists in human tissues and cells. DIR is a key molecule that can adaptively regulate the functions of cells and tissues through high expression and secretion in a hypoxic cellular environment.

Therefore, the inventor hypothesized that DIR was likely to enter the blood through the blood supply system in diseased tissues. Subsequently, the inventor further proved the correctness of this hypothesis through the following experiments: the inventor first transferred DIR plasmids into HEK293 cells, and then detected the presence of a large amount of DIR proteins in the medium, proving that DIR can be secreted into the cells; and afterwards, DIR was subsequently detected in the plasma of AD and MCI patients, while no DIR or very low levels of DIR were detected in the plasma from normal humans and non-AD patients. This result shows that DIR specifically exists in the blood of AD and MCI patients and can be used as a blood marker for AD diagnosis (e.g., early diagnosis); and at the same time, DIR was found increased significantly in the detected blood samples from other patients with cognitive impairment. Therefore, the DIR level in the blood can be used as a new marker in the diagnosis and treatment of AD (presenile dementia).

All numerical ranges provided throughout the present application are intended to clearly comprise all numbers falling between the endpoints of the range and the numerical ranges falling therebetween. Features mentioned in the present application or in the examples can be combined. All features disclosed in the present application can be used in any combination form, and each feature disclosed in the specification can be replaced by any alternative feature capable of providing the same, equivalent or similar purpose. Therefore, unless otherwise stated, the features disclosed are only general examples of equivalent or similar features.

As used in the present application, "comprise", "have" or "include" comprises "comprise", "consists essentially of", "consists substantially of" and "consist of"; and "consists essentially of", "consists substantially of" and "consist of" are subordinate concepts of "comprise", "have" or "include".

In the present application, the neurodegenerative disease may comprise an acute neurodegenerative disease and a chronic neurodegenerative disease. For example, the neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

In the present application, the neurodegenerative disease may comprise presenile dementia. For example, the neurodegenerative disease may comprise early presenile dementia, intermediate presenile dementia and/or late presenile dementia.

In the present application, the cognitive impairment may comprise mild cognitive impairment (MCI), intermediate cognitive impairment and late cognitive impairment. For example, the cognitive impairment may comprise cognitive impairment caused by normal aging, Lewy body dementia (LBD), frontotemporal dementia and/or vascular dementia. For example, the cognitive impairment-inducing diseases may induce diseases comprise Alzheimer's disease, multi-infarct type, Parkinson's disease, AIDS and/or Creutzfeldt-Jakob disease (CJD).

In the present application, the cognitive impairment may comprise mild cognitive impairment (MCI) (e.g., loss of short-term memory, difficulty in expressing or understanding abstract things, erratic mood or behavior, difficulty in learning new things and following complex instructions, impaired judgment and/or needing reminders from others for basic self-care), intermediate cognitive impairment (e.g., confusion between long-term memory and memory of real-life situations, poor expression, changes in behavior or personality, or emotional instability and/or need for assistance in basic self-care) or late cognitive impairment (e.g., memory impairment, decline in physical activity and mental status, inability to express or communicate effectively, inability to take care of oneself, and/or circadian disorder).

In the present application, the cognitive impairment may comprise suffering from a disease that may cause the induction of the cognitive impairment. For example, the subject may suffer from Alzheimer's disease, multi-infarct type, Parkinson's disease, AIDS and/or Creutzfeldt-Jakob disease (CJD).

### Diagnostic Target DIR

In one aspect, the present application provides use of a DNA damage-inducible transcript 4-like (DDIT4L) intron retention spliced product (DIR), and/or a nucleic acid encoding the DIR, in diagnosis of a disease and/or in evaluation of progression of the disease, wherein the disease may comprise cognitive impairment.

In one aspect, the present application provides use of a DNA damage-inducible transcript 4-like (DDIT4L) intron retention spliced product (DIR), and/or a nucleic acid encoding the DIR, in diagnosis of a disease and/or in evaluation of progression of the disease, wherein the disease may comprise a neurodegenerative disease.

DDIT4L (DNA Damage Inducible Transcript 4 Like, SEQ ID NO: 92) is a protein-encoding gene related to an mTOR signal transduction pathway, and its levels in macrophages may be up-regulated by means of oxidized LDL and hypoxia.

After removing an intron from the DDIT4L gene through normal splicing, a nucleotide sequence (SEQ ID NO: 94) encoding a 193aa long DDIT4L protein (SEQ ID NO: 95) may be obtained. However, the applicant has unexpectedly found that there is another special splicing mode for this gene, i.e., intron retention splicing. With this special splicing mode, 2212 nt of nucleotide sequence is obtained, and an 84aa spliced product peptide (SEQ ID NO: 1) is finally obtained since the intron may comprise a terminator. In the present application, the protein obtained by intron retention splicing may be referred to as "QDLIR or DIR" (as shown in FIG.1A).

As used in the present application, unless otherwise stated or expressly identifiable, the term "DIR" may comprise a mature mRNA, spliced peptide encoding molecules or spliced product peptides (or DIR proteins) obtained by intronic retention splicing of the DDIT4L gene.

When the spliced product can be mRNA, it can be selected from: (a) a nucleotide molecule with a sequence as set forth in SEQ ID NO: 93; or (b) a nucleotide molecule with a high sequence identity (e.g., which may be at least 80%, 85%, 90%, 92%, 95%, 98%, 99%, 99.5%, or 99.8%) to the nucleotide molecule of SEQ ID NO: 93.

When the spliced product can be a DIR encoding molecule, it can be selected from: (a) a nucleotide molecule with a sequence as set forth in SEQ ID NO: 8; or (b) a nucleotide molecule with a high sequence identity (e.g., which may be at least 80%, 85%, 90%, 92%, 95%, 98%, 99%, 99.5%, or 99.8%) to the nucleotide molecule of SEQ ID NO: 8.

When the spliced product can be a DIR protein, it can be selected from: (a') a polypeptide molecule with a sequence as set forth in SEQ ID NO: 1; or (b') a protein molecule with a high sequence identity (e.g., which may be at least 80%, 85%, 90%, 92%, 95%, 98%, 99%, 99.5%, or 99.8%) to the polypeptide molecule of SEQ ID NO: 1; (c') a protein molecule translated from any nucleotide molecule in (a) or (b) above; or (d') a conserved variant polypeptide of the protein molecule described in any one of (a') to (c') above, or a homologous protein or polypeptide thereof, or an active fragment thereof.

### Diagnostic Substance and Diagnostic Kit

In another aspect, the present application provides use of a substance for detecting a DNA damage-inducible transcript 4-like DDIT4L intron retention spliced product DIR, and/or a nucleic acid encoding said DIR, in preparation of a product for diagnosis of a disease and/or in evaluation of progression of said disease, wherein said disease may comprise cognitive impairment.

In another aspect, the present application provides use of a substance for detecting a DNA damage-inducible transcript 4-like (DDIT4L) intron retention spliced product (DIR), and/or a nucleic acid encoding the DIR, in preparation of a product for diagnosis of a disease and/or in evaluation of progression of the disease, wherein the disease may comprise a neurodegenerative disease.

As used in the present application, the terms "detection substance", "detection reagent" or "reagent for detecting DIR" or "reagent for detecting DIR expression level" are used interchangeably, and they all refer to substances that specifically target DIR molecules and can be used to directly or indirectly detect the presence and/or content of DIR molecules. These detection substances can detect DIR at the gene level or protein level.

In the present application, the substance may comprise an antigen-binding protein capable of specifically binding to the DIR, a probe capable of specifically binding to the DIR, a primer capable of specifically amplifying the nucleic acid encoding the DIR, a gene chip capable of specifically detecting the nucleic acid encoding the DIR, an aptamer capable of specifically binding to the nucleic acid encoding the DIR, and/or a guide RNA capable of specifically binding to the nucleic acid encoding the DIR.

In the present application, the kit may comprise at least one (e.g., 1, 2, 3 or more) detection substance. For example, the kit may comprise at least one (e.g., 1, 2, 3, or more) antigen-binding protein (e.g., a DIR antibody) capable of specifically binding to DIR. In the present application, different antigen-binding proteins existing in the amino acid sequence of at least one CDR may be considered as different species of antigen-binding proteins.

Depending on the sequence of the DIR molecule, those of ordinary skill in the art can prepare a reagent specifically targeting the DIR molecule based on conventional means or obtain the reagent commercially. For example, the detection reagent that can be used in the present application may include, but are not limited to: antigen-binding proteins (for example, monoclonal antibodies), probes, gene chips, PCR primers, gRNA or others having detection specificity for DIR molecules.

Moreover, for the sake of detection, the detection reagent of the present application may also come with detectable labels, which may include, but are not limited to: radioactive isotopes, fluorophores, chemiluminescent moieties, enzymes, enzyme substrates, enzyme cofactors, enzyme inhibitors, dyes, metal ions, ligands (such as biotins or haptens), etc.

The detection reagent of the present application may exist in a solution, be fixed on a carrier (such as a substrate or an adsorbent), or exist in other conventional modes in the art, as long as its mode of existence is suitable for DIR detection in biological samples. For example, when the detection reagent of the present application can be a nucleotide probe, it may exist in the form of a biochip (or "microarray").

In the present application, the product may exist in a kit form.

The present application further provides a product for diagnosis of presenile dementia and/or evaluation of progression (e.g., a grade or stage) of the presenile dementia. The product may comprise: a substance for detecting a DIR level, and optionally, other substances for diagnosis of presenile dementia and/or evaluation of progression (e.g., a grade or stage) of presenile dementia, for example, detection substances for existing AD markers.

In the present application, the diagnostic kit may comprise an instruction manual recording a method of using the diagnostic kit for diagnosis of cognitive impairment, and/or evaluation of progression of the cognitive impairment; and/or for diagnosis of a neurodegenerative disease, and/or evaluation of progression of the neurodegenerative disease.

According to the needs of the detection method used, an appropriate DIR detection substance may be selected and prepared into a product (such as a kit) suitable for the detection method used. Those of ordinary skill in the art can adjust and change the detection method and the reagent contained in the product according to actual conditions and needs.

Accordingly, the present application further provides a product (such as a detection kit), which may comprise: (i) a detection effective amount of one or more reagents for detecting DIR; and (ii) optionally, one or more substances selected from the group consisting of: a container, an instruction manual, a positive control, a negative control, a buffer, an auxiliary or solvent, for example, a solution for suspending or fixing cells, a detectable label or marker, a solution facilitating hybridization of nucleic acids, a solution for cell lysis, or a solution for nucleic acid purification.

In one example, the present application provides a detection kit suitable for detecting the expression of a DIR protein in a biological sample by immunohistochemistry. The detection kit may comprise: a blocking solution, for example, 10% goat serum; a primary antibody, for example, a rabbit anti-human or mouse DIR monoclonal antibody; a secondary antibody, for example, a labeled (such as HRP labeled) or unlabeled goat anti-rabbit secondary antibody; a substrate buffer, for example, a DAB substrate buffer; a developing solution; and optionally, a container containing the above reagents and an instruction manual.

The diagnostic kit of the present application may also come with an instruction manual of the kit, which records how to detect with the kit, and how to diagnose cognitive impairment and/or evaluate the progression of the cognitive impairment, and/or diagnose a neurodegenerative disease and/or evaluate the progression of the neurodegenerative disease, by using a detection result. For example, the kit described in the present application can be used for diagnosis of AD and/or evaluation of progression (e.g., a grade or stage) of AD.

The product of the present application may further comprise other reagents that are clinically used for diagnosis of AD and/or evaluation of AD progression (e.g., grading or staging) in a subject, in order to assist or verify the results obtained by detecting DIR. Those of ordinary skill in the art can make routine selections based on specific needs.

### Diagnosis Method

In another aspect, the present application provides a method for diagnosing cognitive impairment, and/or evaluating progression of the cognitive impairment. The method may comprise the step of: detecting, from a sample of a subject in need thereof, a content of a DNA damage-inducible transcript 4-like (DDIT4L) intron retention spliced product DIR, and/or of a nucleic acid encoding the DIR.

In another aspect, the present application provides a method for diagnosing a neurodegenerative disease, and/or evaluating progression of the neurodegenerative disease. The method may comprise the step of: detecting, from a sample of a subject in need thereof, a content of a DNA damage-inducible transcript 4-like (DDIT4L) intron retention spliced product DIR, and/or of a nucleic acid encoding the DIR.

In the present application, the subject may comprise a mammal.

In the present application, the subject may comprise a tumor patient.

In the present application, the subject is in an old age stage.

In the present application, the sample may comprise a blood sample and/or a tissue or cell sample.

In the present application, the sample may comprise whole blood, serum, plasma, and/or cerebrospinal fluid.

In the present application, the method may comprise: comparing the content of the DIR and/or of the nucleic acid encoding the DIR in the sample of the subject with a normal control value, wherein the normal control value may comprise a content of the DIR and/or of the nucleic acid encoding the DIR in a normal subject.

In the present application, the method may be used to diagnose cognitive impairment and/or evaluate the progression of the cognitive impairment by using the kit of the present application, and/or the detection substance of the present application.

In the present application, the normal subject may not suffer from the cognitive impairment. The normal subject may not suffer from the neurodegenerative disease.

In the present application, when the content of the DIR and/or of the nucleic acid encoding the DIR in the sample of the subject is significantly higher (for example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more higher) than the normal control value, the subject may be diagnosed with the cognitive impairment and/or the neurodegenerative disease.

In the present application, the method may comprise: comparing the content of the DIR and/or of the nucleic acid encoding the DIR in the sample of the subject with an early control value, wherein the early control value may comprise a previously measured content of the DIR and/or of the nucleic acid encoding the DIR in the same subject.

In the present application, the subject has been diagnosed with the cognitive impairment and/or the neurodegenerative disease. For example, the subject has been diagnosed with the cognitive impairment; or the subject has been diagnosed with the neurodegenerative disease.

In the present application, when the content of the DIR and/or of the nucleic acid encoding the DIR in the sample of the subject is significantly higher (for example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more higher) than the early control value, the subject may be diagnosed with aggravated progression of the cognitive impairment and/or the neurodegenerative disease.

In the present application, the method may further comprise the step of detecting a content of a marker associated with the cognitive impairment and/or the neurodegenerative disease in a sample derived from the subject.

In the present application, the marker associated with the cognitive impairment and/or the neurodegenerative disease may further comprise AD7C-NTP, pTau-181, pTau-217 and /or Aβ1-42. The detection result of the content of the marker may be used to assist the diagnosis of cognitive impairment, and/or the evaluation of progression of the cognitive impairment, and/or for use in diagnosis of a neurodegenerative disease, and/or the evaluation of progression of the neurodegenerative disease.

In the present application, the method may further comprise the step of observing brain imaging of the subject. The result of the brain imaging may be used to assist the diagnosis of cognitive impairment, and/or the evaluation of progression of the cognitive impairment, and/or for use in diagnosis of a neurodegenerative disease, and/or the evaluation of progression of the neurodegenerative disease.

### Diagnosis System

In another aspect, the present application provides a system for diagnosing cognitive impairment, and/or evaluating progression of the cognitive impairment. The system may comprise a substance for detecting a DNA damage-inducible transcript 4-like (DDIT4L) intron retention spliced product (DIR), and/or a nucleic acid encoding the DIR.

In another aspect, the present application provides a system for diagnosing a neurodegenerative disease, and/or evaluating progression of the neurodegenerative disease. The system may comprise a substance for detecting a DNA damage-inducible transcript 4-like (DDIT4L) intron retention spliced product (DIR), and/or a nucleic acid encoding the DIR.

In the present application, the system may comprise a detection module capable of detecting a content value of the DIR and/or of the nucleic acid encoding the DIR.

In the present application, the detection module detects a content value of the DIR and/or of the nucleic acid encoding the DIR in the sample acquired by the collection module.

In the present application, the detection module may comprise a substance capable of detecting a content value of the DIR and/or of the nucleic acid encoding the DIR.

In the present application, the substance may comprise an antigen-binding protein capable of specifically binding to the DIR, a probe capable of specifically binding to the DIR, a primer capable of specifically amplifying the nucleic acid encoding the DIR, a gene chip capable of specifically detecting the nucleic acid encoding the DIR, an aptamer capable of specifically binding to the nucleic acid encoding the DIR, and/or a guide RNA capable of specifically binding to the nucleic acid encoding the DIR.

In the present application, the detection module may comprise at least one (e.g., 1, 2, 3 or more) detection substance. For example, the detection module may comprise at least one (e.g., 1, 2, 3, or more) antigen-binding protein (e.g., a DIR antibody) capable of specifically binding to DIR. In the present application, different antigen-binding proteins existing in the amino acid sequence of at least one CDR may be considered as different species of antigen-binding proteins.

In the present application, the detection module may comprise an instrument capable of detecting a content of the DNA damage-inducible transcript 4-like (DDIT4L) intron retention spliced product (DIR), and/or of the nucleic acid encoding the DIR. For example, the instrument may comprise an instrument required to perform qPCR, ELISA and western blotting, and/or a gene chip.

In the present application, the detection module may comprise a substance and/or an instrument capable of detecting a content of a marker associated with the cognitive impairment and/or the neurodegenerative disease.

In the present application, the marker associated with the cognitive impairment and/or the neurodegenerative disease may further comprise AD7C-NTP, pTau-181, pTau-217 and /or Aβ1-42. For example, the substance may further comprise an antibody capable of specifically binding to the marker, a probe capable of specifically binding to the marker, a primer capable of specifically amplifying the nucleic acid encoding the marker, a gene chip capable of specifically detecting the nucleic acid encoding the marker, an aptamer capable of specifically binding to the nucleic acid encoding the marker, and/or a guide RNA capable of specifically binding to the nucleic acid encoding the marker. For example, the instrument may comprise an instrument required to perform qPCR, ELISA and western blotting, and/or a gene chip.

In the present application, the detection module may output a content value of the DIR and/or of the nucleic acid encoding the DIR.

In the present application, the system may comprise a determination module capable of determining a diagnosis result and/or a disease progression result of said subject based on the content of said DIR and/or of said nucleic acid encoding said DIR as well as said normal control value and/or said early control value; the normal control value may comprise a content of the DIR and/or of the nucleic acid encoding the DIR in a normal subject; and wherein the early control value may comprise a previously measured content of the DIR and/or of the nucleic acid encoding the DIR in the same subject.

In the present application, the "previously" may include at least 10 days ago, at least 20 days ago, at least 1 month ago, at least 6 months ago, at least 12 months ago, at least 24 months ago or more ago. In the present application, the subject may have been diagnosed with the cognitive impairment and/or the neurodegenerative disease. With the system of the present application, the content of the DIR and/or of the nucleic acid encoding the DIR in the subject at different time stages may be traced, thereby helping to diagnose the progression of the cognitive impairment and/or of the neurodegenerative disease of the subject.

In the present application, the normal subject may not suffer from the cognitive impairment and/or the neurodegenerative disease.

For example, when the content of the DIR and/or of the nucleic acid encoding the DIR in the sample of the subject is significantly higher (for example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more higher) than the normal control value, the determination module may diagnose that the subject may suffer from the cognitive impairment and/or the neurodegenerative disease.

For example, when the content of the DIR and/or of the nucleic acid encoding the DIR in the sample of the subject is significantly higher (for example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more higher) than the early control value, the determination module may diagnose that the subject may have aggravated progression of the cognitive impairment and/or the neurodegenerative disease.

In the present application, the determination module may output a diagnosis result of the subject.

In the present application, the system may comprise a display module capable of displaying the diagnosis result of the determination module. For example, the diagnostic results displayed by the display module may comprise qualitative and/or quantitative results.

In the present application, the system may comprise a storage module capable of storing the content value output by the detection module, and/or the diagnosis result output by the determination module.

In the present application, the system may comprise the following devices:
a) a device for collecting and/or receiving data on a DIR level in a sample; and
b) a device for analyzing the data for diagnosis of AD and/or for evaluation of progression of AD in the subject, wherein DIR being higher than a control level indicates the subject suffers from AD or undergoes some development of AD.

In the present application, the system may further comprise (a') a device for collecting and/or receiving AD diagnosis and/or data on other clinical indicators or basic characteristics of the development of the AD in a sample.

In the present application, the device described in (b) is used to combine and analyze DIR level data with data on other clinical indicators or basic characteristics of AD diagnosis and/or development progression of AD, in order to perform prognosis evaluation on the AD diagnosis and/or AD development progression of the subject.

Or, in the present application, the system may further comprise a device for analyzing AD diagnosis and/or data on other clinical indicators or basic characteristics of the progression of AD; and/or a device for combining and analyzing DIR level data with data on other clinical indicators or basic characteristics of AD diagnosis and/or development progression of AD, in order to evaluate the AD diagnosis and/or the progression of AD of the subject.

In the present application, the system may further comprise one or more devices selected from the group consisting of: a device for inputting and/or outputting and/or storing data; a device for outputting and/or storing analysis results, for example, a device for uploading and/or storing the analysis results to a cloud database or its corresponding cloud database device; and a device for performing intelligent analysis and recommending a therapeutic scheme based on evaluation results.

It should be understood that those skilled in the art may add other conventional devices or programs to the system as needed.

Generally, the diagnosis of AD and/or the evaluation of progression (e.g., grading or staging) of AD may be performed with a method including: detecting the level of DIR molecules in a subject or a sample derived from the subject, and comparing the level with a control level; and if a comparison result shows that the level of DIR molecules in the subject is higher than the control level, providing a prompt that the subject suffers from AD or has some development of AD. In some embodiments, the method of the present application optionally further comprises: obtaining a sample to be tested from the subject; and contacting the sample to be tested with a reagent or kit for detecting the DIR level.

As used herein, the term "control" may comprise a normal control or a control with a specific developmental grade or stage of AD. "Control" may refer to the level of a DIR molecule used as a reference, including but not limited to, a DIR molecule level measured in a non-AD normal biological sample from the same subject (e.g., a sample derived from the subject who is not an AD patient or is a normal human), a statistically determined population standard level, or a standardized level. The term "control with a specific developmental grade or stage of AD" refers to a DIR molecule level measured in the same subject at a previously determined developmental grade or stage, a statistically determined population standard level at that grade or stage, or a normalized s level.

### Antigen-Binding Protein of DIR

In the present application, the antigen-binding protein may comprise LCDR2, which may comprise the amino acid sequence as set forth in SEQ ID NO: 74. For example, the antigen-binding protein of the present application may have DIR binding capability. For example, the CDR may be determined by the IMGT numbering scheme.

X₁ X₂S (SEQ ID NO.74), with X₁ being A or Y and X₂ being A or Y.

In the present application, the LCDR2 may comprise the amino acid sequence as set forth in SEQ ID NO: 26 or 16. The amino acid sequence of SEQ ID NO: 16 is YTS, and the amino acid sequence of SEQ ID NO: 26 is AAS.

In the present application, the antigen-binding protein may comprise LCDR1, which may comprise the amino acid sequence as set forth in SEQ ID NO: 73. For example, the antigen-binding protein of the present application may have DIR binding capability. For example, the CDR may be determined by the IMGT numbering scheme. For example, the LCDR1 may comprise LCDR1s in the light chain variable regions of IR-II-1 to IR-II-10.

Q X₁ X₂D X₃ X₄ X₅ X₆ X₇Y (SEQ ID NO.73), with X₁ being absent or S, X₂ being absent or V, X₃ being absent or Y, X₄ being absent or D, X₅ being G or I, X₆ being D, E or S, and X₇ being N or S.

In the present application, the LCDR1 may comprise the amino acid sequence as set forth in any one of SEQ ID NOs: 25, 52 and 15.

In the present application, the antigen-binding protein may comprise LCDR3, which may comprise the amino acid sequence as set forth in SEQ ID NO: 75. For example, the antigen-binding protein of the present application may have DIR binding capability. For example, the CDR may be determined by the IMGT numbering scheme. For example, the LCDR3 may comprise LCDR3s in the light chain variable regions of IR-II-1 to IR-II-10.

X₁₀ X₂ X₃ X₄ X₅P X₆ X₇ (SEQ ID NO.75), with X₁ being L or Q, X₂ being S or Y, X₃ being N, S or Y, X₄ being D, E or K, X₅ being D or L, X₆ being F or R, and X₇ being A or T.

In the present application, the LCDR3 may comprise the amino acid sequence as set forth in any one of SEQ ID NOs: 27, 53, 67 and 17.

In the present application, the antigen-binding protein may comprise LCDR3, LCDR2 and LCDR1. For example, in the antigen-binding protein, the LCDR2 may comprise the amino acid sequence as set forth in SEQ ID NO: 74, the LCDR1 may comprise the amino acid sequence as set forth in SEQ ID NO: 73, and the LCDR3 may comprise the amino acid sequence as set forth in SEQ ID NO: 75. For example, the antigen-binding protein of the present application may have DIR binding capability. For example, the CDR may be determined by the IMGT numbering scheme. For example, the LCDR1 to LCDR3 may comprise LCDR1s to LCDR3s in the light chain variable regions of IR-II-1 to IR-II-10.

In the present application, the antigen-binding protein may comprise LCDR3, LCDR2 and LCDR1. For example, in the antigen-binding protein, the LCDR1 may comprise the amino acid sequence as set forth in SEQ ID NO: 25, the LCDR2 may contain the amino acid sequence as set forth in SEQ ID NO: 26, and the LCDR3 may comprise the amino acid sequence as set forth in SEQ ID NO: 27.

For example, in the antigen-binding protein, the LCDR1 may comprise the amino acid sequence as set forth in SEQ ID NO: 52, the LCDR2 may contain the amino acid sequence as set forth in SEQ ID NO: 26, and the LCDR3 may comprise the amino acid sequence as set forth in SEQ ID NO: 53.

For example, in the antigen-binding protein, the LCDR1 may comprise the amino acid sequence as set forth in SEQ ID NO: 52, the LCDR2 may contain the amino acid sequence as set forth in SEQ ID NO: 26, and the LCDR3 may comprise the amino acid sequence as set forth in SEQ ID NO: 67.

For example, in the antigen-binding protein, the LCDR1 may comprise the amino acid sequence as set forth in SEQ ID NO: 15, the LCDR2 may comprise the amino acid sequence as set forth in SEQ ID NO: 16, and the LCDR3 may comprise the amino acid sequence as set forth in SEQ ID NO: 17.

For example, the antigen-binding protein of the present application may have DIR binding capability. For example, the CDR may be determined by the IMGT numbering scheme. For example, the LCDR1 to LCDR3 may comprise LCDR1s to LCDR3s in the heavy chain variable regions of IR-II-1 to IR-II-10.

In the present application, the antigen-binding protein may comprise HCDR1, which may comprise the amino acid sequence as set forth in SEQ ID NO: 70. For example, the antigen-binding protein of the present application may have DIR binding capability. For example, the CDR may be determined by the IMGT numbering scheme. For example, the HCDR1 may comprise HCDR1s in the heavy chain variable regions of IR-II-1 to IR-II-10.

GYTFX₁X₂YX₃ (SEQ ID NO.70), with X₁ being S or T, X₂ being E, N, R or S, X₃ being T or W.

In the present application, the HCDR1 may comprise the amino acid sequence as set forth in any one of SEQ ID NOs: 20, 35, 56 and 10.

In the present application, the antigen-binding protein may comprise HCDR2, which may comprise the amino acid sequence as set forth in SEQ ID NO: 71. For example, the antigen-binding protein of the present application may have DIR binding capability. For example, the CDR may be determined by the IMGT numbering scheme. For example, the HCDR1 may comprise HCDR2s in the heavy chain variable regions of IR-II-1 to IR-II-10.

IX₁P X₂ X₃ X₄ X₅ X₆T (SEQ ID NO.71), with X₁ being L or Y, X₂ being G or H, X₃ being G, N or S, X₄ being absent or Y, X₅ being G or Y, X₆ being G, N, S or T.

In the present application, the HCDR2 may comprise the amino acid sequence as set forth in any one of SEQ ID NOs: 21, 36, 42, 48 and 11.

In the present application, the antigen-binding protein may comprise HCDR3, which may comprise the amino acid sequence as set forth in SEQ ID NO: 72. For example, the antigen-binding protein of the present application may have DIR binding capability. For example, the CDR may be determined by the IMGT numbering scheme. For example, the HCDR3 may comprise HCDR3s in the heavy chain variable regions of IR-II-1 to IR-II-10.

X₁ X₂ X₃ X₄ X₅ X₆ X₇ X₈ X₉ X₁₀ X₁₁ X₁₂ X₁₃ X₁₄ X₁₅DY (SEQ ID NO.72), with X₁ being absent or A, X₂ being absent or R, X₃ being absent , S or T, X₄ being absent , D, G or Y, X₅ being absent , D, I, M or V, X₆ being absent , G or I, X₇ being absent , T or Y, X₈ being absent , A, L or T, X₉ being absent , R, S or T, X₁₀ being absent , G or T, X₁₁ being absent , D or E, X₁₂ being D or Y, X₁₃ being F, L or Y, X₁₄ being A, T or V, X₁₅ being F or M.

In the present application, the HCDR3 may comprise the amino acid sequence as set forth in any one of SEQ ID Nos: 22, 30, 37, 43, 49, 57, 62, and 12.

In the present application, the antigen-binding protein may comprise HCDR3, HCDR2 and HCDR1. For example, in the antigen-binding protein, the HCDR2 may comprise the amino acid sequence as set forth in SEQ ID NO: 71, the HCDR1 may comprise the amino acid sequence as set forth in SEQ ID NO: 70, and the HCDR3 may comprise the amino acid sequence as set forth in SEQ ID NO: 72. For example, the antigen-binding protein of the present application may have DIR binding capability. For example, the CDR may be determined by the IMGT numbering scheme. For example, the HCDR1 to HCDR3 may comprise HCDR1s to HCDR3s in the heavy chain variable regions of IR-II-1 to IR-II-10.

For example, in the antigen-binding protein, the HCDR1 may comprise the amino acid sequence as set forth in SEQ ID NO: 20, the HCDR2 may comprise the amino acid sequence as set forth in SEQ ID NO: 21, and the HCDR3 may comprise the amino acid sequence as set forth in SEQ ID NO: 22.

For example, in the antigen-binding protein, the HCDR1 may comprise the amino acid sequence as set forth in SEQ ID NO: 20, the HCDR2 may comprise the amino acid sequence as set forth in SEQ ID NO: 21, and the HCDR3 may comprise the amino acid sequence as set forth in SEQ ID NO: 30.

For example, in the antigen-binding protein, the HCDR1 may comprise the amino acid sequence as set forth in SEQ ID NO: 35, the HCDR2 may comprise the amino acid sequence as set forth in SEQ ID NO: 36, and the HCDR3 may comprise the amino acid sequence as set forth in SEQ ID NO: 37.

For example, in the antigen-binding protein, the HCDR1 may comprise the amino acid sequence as set forth in SEQ ID NO: 20, the HCDR2 may comprise the amino acid sequence as set forth in SEQ ID NO: 42, and the HCDR3 may comprise the amino acid sequence as set forth in SEQ ID NO: 43.

For example, in the antigen-binding protein, the HCDR1 may comprise the amino acid sequence as set forth in SEQ ID NO: 20, the HCDR2 may comprise the amino acid sequence as set forth in SEQ ID NO: 48, and the HCDR3 may comprise the amino acid sequence as set forth in SEQ ID NO: 49.

For example, in the antigen-binding protein, the HCDR1 may comprise the amino acid sequence as set forth in SEQ ID NO: 56, the HCDR2 may comprise the amino acid sequence as set forth in SEQ ID NO: 36, and the HCDR3 may comprise the amino acid sequence as set forth in SEQ ID NO: 57.

For example, in the antigen-binding protein, the HCDR1 may comprise the amino acid sequence as set forth in SEQ ID NO: 35, the HCDR2 may comprise the amino acid sequence as set forth in SEQ ID NO: 36, and the HCDR3 may comprise the amino acid sequence as set forth in SEQ ID NO: 62.

For example, in the antigen-binding protein, the HCDR1 may comprise the amino acid sequence as set forth in SEQ ID NO: 35, the HCDR2 may comprise the amino acid sequence as set forth in SEQ ID NO: 42, and the HCDR3 may comprise the amino acid sequence as set forth in SEQ ID NO: 37.

For example, in the antigen-binding protein, the HCDR1 may comprise the amino acid sequence as set forth in SEQ ID NO: 56, the HCDR2 may comprise the amino acid sequence as set forth in SEQ ID NO: 36, and the HCDR3 may comprise the amino acid sequence as set forth in SEQ ID NO: 57.

For example, in the antigen-binding protein, the HCDR1 may comprise the amino acid sequence as set forth in SEQ ID NO: 10, the HCDR2 may comprise the amino acid sequence as set forth in SEQ ID NO: 11, and the HCDR3 may comprise the amino acid sequence as set forth in SEQ ID NO: 12.

For example, the antigen-binding protein of the present application may have DIR binding capability. For example, the CDR may be determined by the IMGT numbering scheme. For example, the HCDR1 to HCDR3 may comprise HCDR1s to HCDR3s in the heavy chain variable regions of IR-II-1 to IR-II-10.

In the present application, the antigen-binding protein may comprise HCDR3, HCDR2, HCDR1, LCDR3, LCDR2 and LCDR1.

For example, in the antigen-binding protein of the present application, the HCDR1 may comprise the amino acid sequence as set forth in SEQ ID NO: 20, the HCDR2 may comprise the amino acid sequence as set forth in SEQ ID NO: 21, the HCDR3 may comprise the amino acid sequence as set forth in SEQ ID NO: 22, the LCDR1 may comprise the amino acid sequence as set forth in SEQ ID NO: 25, the LCDR2 may comprise the amino acid sequence as set forth in SEQ ID NO: 26, the LCDR3 may comprise the amino acid sequence as set forth in SEQ ID NO: 27.

For example, in the antigen-binding protein of the present application, the HCDR1 may comprise the amino acid sequence as set forth in SEQ ID NO: 20, the HCDR2 may comprise the amino acid sequence as set forth in SEQ ID NO: 21, the HCDR3 may comprise the amino acid sequence as set forth in SEQ ID NO: 30, the LCDR1 may comprise the amino acid sequence as set forth in SEQ ID NO: 25, the LCDR2 may comprise the amino acid sequence as set forth in SEQ ID NO: 26, the LCDR3 may comprise the amino acid sequence as set forth in SEQ ID NO: 27.

For example, in the antigen-binding protein of the present application, the HCDR1 may comprise the amino acid sequence as set forth in SEQ ID NO: 35, the HCDR2 may comprise the amino acid sequence as set forth in SEQ ID NO: 36, the HCDR3 may comprise the amino acid sequence as set forth in SEQ ID NO: 37, the LCDR1 may comprise the amino acid sequence as set forth in SEQ ID NO: 25, the LCDR2 may comprise the amino acid sequence as set forth in SEQ ID NO: 26, the LCDR3 may comprise the amino acid sequence as set forth in SEQ ID NO: 27.

For example, in the antigen-binding protein of the present application, the HCDR1 may comprise the amino acid sequence as set forth in SEQ ID NO: 20, the HCDR2 may comprise the amino acid sequence as set forth in SEQ ID NO: 42, the HCDR3 may comprise the amino acid sequence as set forth in SEQ ID NO: 43, the LCDR1 may comprise the amino acid sequence as set forth in SEQ ID NO: 25, the LCDR2 may comprise the amino acid sequence as set forth in SEQ ID NO: 26, the LCDR3 may comprise the amino acid sequence as set forth in SEQ ID NO: 27.

For example, in the antigen-binding protein of the present application, the HCDR1 may comprise the amino acid sequence as set forth in SEQ ID NO: 20, the HCDR2 may comprise the amino acid sequence as set forth in SEQ ID NO: 48, the HCDR3 may comprise the amino acid sequence as set forth in SEQ ID NO: 49, the LCDR1 may comprise the amino acid sequence as set forth in SEQ ID NO: 52, the LCDR2 may comprise the amino acid sequence as set forth in SEQ ID NO: 26, the LCDR3 may comprise the amino acid sequence as set forth in SEQ ID NO: 53.

For example, in the antigen-binding protein of the present application, the HCDR1 may comprise the amino acid sequence as set forth in SEQ ID NO: 56, the HCDR2 may comprise the amino acid sequence as set forth in SEQ ID NO: 36, the HCDR3 may comprise the amino acid sequence as set forth in SEQ ID NO: 57, the LCDR1 may comprise the amino acid sequence as set forth in SEQ ID NO: 25, the LCDR2 may comprise the amino acid sequence as set forth in SEQ ID NO: 26, the LCDR3 may comprise the amino acid sequence as set forth in SEQ ID NO: 27.

For example, in the antigen-binding protein of the present application, the HCDR1 may comprise the amino acid sequence as set forth in SEQ ID NO: 35, the HCDR2 may comprise the amino acid sequence as set forth in SEQ ID NO: 36, the HCDR3 may comprise the amino acid sequence as set forth in SEQ ID NO: 62, the LCDR1 may comprise the amino acid sequence as set forth in SEQ ID NO: 25, the LCDR2 may comprise the amino acid sequence as set forth in SEQ ID NO: 26, the LCDR3 may comprise the amino acid sequence as set forth in SEQ ID NO: 27.

For example, in the antigen-binding protein of the present application, the HCDR1 may comprise the amino acid sequence as set forth in SEQ ID NO: 35, the HCDR2 may comprise the amino acid sequence as set forth in SEQ ID NO: 42, the HCDR3 may comprise the amino acid sequence as set forth in SEQ ID NO: 37, the LCDR1 may comprise the amino acid sequence as set forth in SEQ ID NO: 52, the LCDR2 may comprise the amino acid sequence as set forth in SEQ ID NO: 26, the LCDR3 may comprise the amino acid sequence as set forth in SEQ ID NO: 53.

For example, in the antigen-binding protein of the present application, the HCDR1 may comprise the amino acid sequence as set forth in SEQ ID NO: 56, the HCDR2 may comprise the amino acid sequence as set forth in SEQ ID NO: 36, the HCDR3 may comprise the amino acid sequence as set forth in SEQ ID NO: 57, the LCDR1 may comprise the amino acid sequence as set forth in SEQ ID NO: 52, the LCDR2 may comprise the amino acid sequence as set forth in SEQ ID NO: 26, the LCDR3 may comprise the amino acid sequence as set forth in SEQ ID NO: 67.

For example, in the antigen-binding protein of the present application, the HCDR1 may comprise the amino acid sequence as set forth in SEQ ID NO: 10, the HCDR2 may comprise the amino acid sequence as set forth in SEQ ID NO: 11, the HCDR3 may comprise the amino acid sequence as set forth in SEQ ID NO: 12, the LCDR1 may comprise the amino acid sequence as set forth in SEQ ID NO: 15, the LCDR2 may contain the amino acid sequence as set forth in SEQ ID NO: 16, and the LCDR3 may comprise the amino acid sequence as set forth in SEQ ID NO: 17.

For example, the antigen-binding protein of the present application may have DIR binding capability. For example, the CDR may be determined by the IMGT numbering scheme. For example, the HCDR1 to HCDR3 may comprise HCDR1s to HCDR3s in the heavy chain variable regions of IR-II-1 to IR-II-10; and the LCDR1 to LCDR3 may comprise LCDR1s to LCDR3s in the light chain variable regions of IR-II-1 to IR-II-10.

In the present application, the antigen-binding protein may comprise H-FR1, and a C-terminus of the H-FR1 may be directly or indirectly linked to an N-terminus of the HCDR1. In the present application, the antigen-binding protein may comprise H-FR2, which may be located between the HCDR1 and the HCDR2. In the present application, the antigen-binding protein may comprise H-FR3, which may be located between the HCDR2 and the HCDR3. In the present application, the antigen-binding protein may comprise H-FR4, and an N-terminus of the H-FR4 may be linked to a C-terminus of the HCDR3. In the present application, the antigen-binding protein may comprise H-FR1, H-FR2, H-FR3 and H-FR4.

In the present application, the antigen-binding protein may comprise L-FR1, and a C-terminus of the L-FR1 may be directly or indirectly linked to an N-terminus of the LCDR1. In the present application, the antigen-binding protein may comprise L-FR1, and a C-terminus of the L-FR1 may be directly or indirectly linked to an N-terminus of the LCDR1. In the present application, the antigen-binding protein may comprise L-FR2, which may be located between the LCDR1 and the LCDR2. In the present application, the antigen-binding protein may comprise L-FR3, which may be located between the LCDR2 and the LCDR3. In the present application, the antigen-binding protein may comprise L-FR4, and an N-terminus of the L-FR4 may be linked to a C-terminus of the LCDR3. In the present application, the antigen-binding protein may comprise L-FR1, L-FR2, L-FR3 and L-FR4.

In the present application, the antigen-binding protein may comprise a heavy chain variable region VH, which may comprise the amino acid sequence as set forth in any one of SEQ ID NOs: 13, 23, 31, 38, 44, 50, 58, 63 and 65. For example, the antigen-binding protein of the present application may have DIR binding capability.

In the present application, the antigen-binding protein may comprise a light chain variable region VL, which may comprise the amino acid sequence as set forth in any one of SEQ ID NOs: 18, 28, 33, 40, 46, 54, 60 and 68. For example, the antigen-binding protein of the present application may have DIR binding capability.

In the present application, the antigen-binding protein may comprise the VH and the VL. In some embodiments, the VH may comprise the amino acid sequence as set forth in SEQ ID NO: 23, and the VL may comprise the amino acid sequence as set forth in SEQ ID NO: 28.

In some embodiments, the VH may comprise the amino acid sequence as set forth in SEQ ID NO: 31 and the VL may comprise the amino acid sequence as set forth in SEQ ID NO: 33.

In some embodiments, the VH may comprise the amino acid sequence as set forth in SEQ ID NO: 38 and the VL may comprise the amino acid sequence as set forth in SEQ ID NO: 40.

In some embodiments, the VH may comprise the amino acid sequence as set forth in SEQ ID NO: 44 and the VL may comprise the amino acid sequence as set forth in SEQ ID NO: 46.

In some embodiments, the VH may comprise the amino acid sequence as set forth in SEQ ID NO: 50 and the VL may comprise the amino acid sequence as set forth in SEQ ID NO: 54.

In some embodiments, the VH may comprise the amino acid sequence as set forth in SEQ ID NO: 58, and the VL may comprise the amino acid sequence as set forth in SEQ ID NO: 60.

In some embodiments, the VH may comprise the amino acid sequence as set forth in SEQ ID NO: 63, and the VL may comprise the amino acid sequence as set forth in SEQ ID NO: 60.

In some embodiments, the VH may comprise the amino acid sequence as set forth in SEQ ID NO: 65, and the VL may comprise the amino acid sequence as set forth in SEQ ID NO: 54.

In some embodiments, the VH may comprise the amino acid sequence as set forth in SEQ ID NO: 58, and the VL may comprise the amino acid sequence as set forth in SEQ ID NO: 68.

In the present application, the VH may comprise the amino acid sequence as set forth in SEQ ID NO: 13, and the VL may comprise the amino acid sequence as set forth in SEQ ID NO: 18. For example, the antigen-binding protein of the present application may have DIR binding capability.

In the present application, the antigen-binding protein may comprise at least one CDR in the VH of the present application. In the present application, the antigen-binding protein may comprise at least one CDR in the VL of the present application. The CDR may be obtained by partitioning in any partitioning mode. In the present application, the CDR may encompass a CDR sequence obtained by partitioning in any CDR partitioning mode, as well as variations thereof.

In the present application, the antigen-binding protein may comprise HCDR1, HCDR2 and HCDR3 in the VH described in the present application.

In the present application, the antigen-binding protein may comprise HCDR1, HCDR2 and HCDR3 in the VH described in the present application. The VH may comprise the amino acid sequence as set forth in any one of SEQ ID NOs: 13, 23, 31, 38, 44, 50, 58, 63, and 65. For example, the antigen-binding protein of the present application may have DIR binding capability. In the present application, the CDR may encompass a CDR sequence obtained by partitioning in any CDR partitioning mode, as well as variations thereof.

In the present application, the antigen-binding protein may comprise LCDR1, LCDR2 and LCDR3 in the VL described in the present application. The VL may comprise the amino acid sequence as set forth in any one of SEQ ID NOs: 18, 28, 33, 40, 46, 54, 60 and 68. For example, the antigen-binding protein of the present application may have DIR binding capability. In the present application, the CDR may encompass a CDR sequence obtained by partitioning in any CDR partitioning mode, as well as variations thereof.

In the present application, the antigen-binding protein may comprise an antibody heavy chain constant region. The antibody heavy chain constant region may be derived from a human IgG, IgA, IgD, IgE and/or IgM heavy chain constant region(s). The antibody heavy chain constant region may be derived from a human IgG heavy chain constant region. In some embodiments, the antigen-binding protein may comprise an antibody heavy chain constant region, which may be derived from a human IgG1, IgG2, IgG3 and/or IgG4 heavy-chain constant region(s). In some embodiments, the antigen-binding protein may comprise an antibody heavy chain constant region, which may be derived from a human IgG1 heavy chain constant region.

In the present application, the antigen-binding protein may comprise an antibody light chain constant region. The antibody light chain constant region may comprise an Igκ-derived constant region or an Igλ-derived constant region. The antibody light chain constant region may be derived from a human Igx constant region.

In the present application, the antigen-binding protein may comprise an antibody or an antigen-binding fragment thereof.

In some embodiments, the antigen-binding fragment may comprise Fab, Fab', a Fv fragment, F(ab')2, F(ab)2, scFv, di-scFv, VHH and/or dAb.

In some embodiments, the antibody may comprise a monoclonal antibody. In the present application, the antibody may comprise a murine antibody and/or a chimeric antibody.

In addition, it should be noted that the antigen-binding protein of the present application may comprise a heavy and/or light chain sequence with one or more conserved sequence modifications. The so-called "conserved sequence modifications" refers to amino acid modifications that would not significantly affect or alter the binding properties of an antibody. Such conserved modifications comprise substitutions, additions, and deletions of amino acids. These modifications may be introduced into the antigen-binding protein of the present application by standard techniques known in the art, for example, point mutation and PCR-mediated mutation. Conserved amino acid substitutions refer to substitutions of amino acid residues with those having similar side chains. There are sets of amino acid residues having similar side chains known in the art. These sets of amino acid residues comprise amino acids with basic side chains (for example, lysine, arginine, and histidine), acidic side chains (for example, aspartic acid, and glutamic acid), uncharged polar side chains (for example, glycine, asparagine, glutamine, serine, threonine, tyrosine, cysteine, and tryptophan), non-polar side chains (for example, alanine, valine, leucine, isoleucine, proline, phenylalanine, and methionine), β-branched side chains (for example, threonine, valine, and isoleucine), and aromatic side chains (for example, tyrosine, phenylalanine, tryptophan, and histidine). In some embodiments, one or more amino acid residues in the CDR of the antigen-binding protein of the present application may be substituted with other amino acid residues in the same side chain set. Those skilled in the art know that some conserved sequence modifications do not abolish antigen binding. For details, see, for example, Brummell et al., (1993) Biochem 32:1180-8; de Wildt et al., (1997) Prot. Eng. 10:835-41; Komissarov et al., (1997), J. Biol. Chem. 272:26864-26870; Hall et al., (1992) J. Immunol. 149:1605-12; Kelley and O'Connell (1993) Biochem. 32:6862-35; Adib-Conquy et al., (1998) Int. Immunol. 10:341-6; and Beers et al., (2000) Clin. Can. Res. 6:2835-43.

The antigen-binding protein described herein can be identified, screened, or characterized by various assays known in the art.

For example, the antigen-binding activity of the antigen-binding protein or a fusion protein described in the present application can be tested by known methods such as enzyme-linked immunosorbent assay (ELISA), immunoblotting (e.g., Western blotting), flow cytometry (e.g., FACS), immunohistochemistry, immunofluorescence, etc.

In the present application, the antigen-binding protein may specifically bind to DIR or a functionally active fragment thereof. In the present application, the antigen-binding protein may specifically bind to DIR-II.

In some embodiments, the DIR or a functionally active fragment thereof may be a full-length DIR or a functionally active fragment thereof, or a fragment exerting the functional activity of DIR (for example, it may be DIR-II). In some embodiments, the DIR or a functionally active fragment thereof may be isolated DIR or a functionally active fragment thereof, or a mixture of various forms of DIR or functionally active fragments thereof. For example, the DIR or a functionally active fragment thereof may be human DIR or a functionally active fragment thereof.

In some embodiments, the binding activity of the antigen-binding protein to an antigen (e.g., DIR-II) may be determined by Elisa. For example, the antigen-binding protein may have the capability of binding to the antigen or the functional fragment thereof at a value of about 10 ng/ml or above (e.g., at least 15 ng/ml, at least 20 ng/ml, at least 25 ng/ml, at least 30 ng/ml, at least 35 ng/ml, at least 40 ng/ml, at least 45 ng/ml, at least 50 ng/ml, at least 55 ng/ml, at least 60 ng/ml, at least 65 ng/ml, at least 70 ng/ml, at least 75 ng/ml, at least 80 ng/ml, at least 85 ng/ml, at least 90 ng/ml, at least 95 ng/ml, at least 100 ng/ml, at least 500 ng/ml, at least 1000 ng/ml or above).

### Medicament Screening Method

In another aspect, the present application provides a medicament screening method, which may comprise the step of: detecting changes in a content of DIR and/or of a nucleic acid encoding the DIR in a subject after administration of a candidate medicament, wherein the medicament is for use in prevention, treatment and/or alleviation of cognitive impairment and/or a neurodegenerative disease.

In the present application, when the content of the DIR and/or of the nucleic acid encoding the DIR in the subject is reduced due to administration of the candidate medicament, the candidate medicament has a therapeutic effect.

In the present application, the administration may comprise injection.

In the present application, the medicament may comprise a small molecule medicament and/or a bio-macromolecular medicament.

According to the disclosure in the present application, the DIR level is closely related to the presence and/or degree of cognitive impairment, and thus can be used as an indicator for diagnosis of AD diagnosis and/or evaluation of the progression (e.g., grading or staging) of AD.

In addition, the present application further provides a method for screening of a candidate medicament for treatment of AD. The method comprises: detecting an effect of the candidate medicament on the DIR level in a subject or a sample derived from the subject, wherein after the use of the candidate medicament, a decrease in the DIR level indicates that the candidate medicament has a therapeutic effect on the AD. Each feature involved in the method for screening of a candidate medicament of the present application may be defined or described as in the present application. In some embodiments, the candidate medicament may be a DIR inhibitor.

The present application found that DIR is specifically expressed in blood samples and/or cerebrospinal fluid of AD patients. One important thing is that DIR can be detected in a small amount of serum (such as about 100 µl), which provides a promising detection means for early diagnosis of AD for clinical application. The current results suggest that the positive detection rates of diseases such as AD and MCI are above 90%, and DIR has a great application prospect as a specific marker for early AD screening.

pTau-181, pTau-217 and Aβ1-42 currently used in clinical blood detection are newly developed detection items for early AD screening. They have relatively low specificity and are expensive. Compared with these existing detections for early AD screening, the early AD screening based on the DIR level in the present application has a high detection rate, very good specificity, and/or low cost, presenting a price advantage. For example, the cost in detection of the DIR level by ELISA may be controlled at less than 10 yuan/person. Therefore, the detection indicators and method of the present application have greater effects and cost advantages compared with existing methods.

In addition, the present application has found that DIR shows good specificity and sensitivity for diagnosis of cognitive impairment and/or a neurodegenerative disease, and can pick out potential AD and/or MCI patients in case of less significant differences among pTau-181, pTau-217, Aβ1-42 and other targets, allowing for effective diagnosis of patients at an earlier stage. In the present application, the kit of the present application and/or the detection substance of the present application may be used in the method to detect changes in the content of DIR and/or of a nucleic acid encoding the DIR in a subject before and/or after the administration of the candidate medicament. In the present application, at least one (e.g., 1, 2, 3 or more) detection substance may be used in the method to detect changes in the content of DIR and/or of a nucleic acid encoding the DIR in a subject before and/or after the administration of the candidate medicament. For example, at least one (e.g., 1, 2, 3 or more) antigen-binding protein (e.g., DIR antibody) capable of specifically binding to DIR may be used in the method. In the present application, different antigen-binding proteins existing in the amino acid sequence of at least one CDR may be considered as different species of antigen-binding proteins.

Information of Sequence Listing:

| **SEQ ID NO:** | Sequence name | Length |
|---|---|---|
| 92 | Full-length sequence of *DDIT4L* gene | 4599 nt |
| 93 | DIR spliced mature mRNA | 4163 nt |
| 8 | DIR protein encoding molecule | 255 bp |
| 1 | DIR protein | 84 aa |
| 94 | DDIT4L protein encoding molecule | 582 nt |
| 95 | DDIT4L protein | 193 aa |

The present application provides the embodiment:
1. Application of a substance for detecting a DNA damage-inducible transcript 4-like (DDIT4L) intron retention spliced product (DIR) from a sample of a subject in preparation of a product (e.g., a kit or detection system) for diagnosis of presenile dementia and mild cognitive impairment and/or evaluation of progression (e.g., grading or staging) of the presenile dementia in the subject.
2. The application according to Embodiment 1, wherein the spliced product is selected from the group consisting of: mRNA having a nucleotide sequence as set forth in SEQ ID NO: 93; a peptide-encoding nucleic acid of the spliced product having a nucleotide sequence as set forth in SEQ ID NO: 8; or a peptide of the spliced product having the amino acid sequence as set forth in SEQ ID NO: 1.
3. The application according to Embodiment 1, wherein the presenile dementia comprises: an early stage (an amnesia stage), an intermediate stage (a neurological disorder stage) and a late stage (a severe dementia stage).
4. The application according to Embodiment 1, wherein the substance is selected from a reagent, an apparatus, or their combination for detecting the DIR.
5. The application according to Embodiment 1, wherein the substance is selected from: a substance specific to the DIR, for example, an antibody (preferably, a monoclonal antibody) of the DIR; a probe, gene chip, PCR primer, or gRNA specific to DIR, etc.
6. The application according to Embodiment 1, wherein the sample is selected from: a blood sample, such as whole blood, serum, and plasma; and cerebrospinal fluid.
7. The application according to Embodiment 1, wherein an increase in the DIR level in the sample compared to the normal control value indicates that the subject suffers from the presenile dementia or mild cognitive impairment; or, an increase in the DIR level in the sample compared to a mild cognitive impairment control or an early control level in a subject who has been diagnosed with presenile dementia indicates some development (e.g., a higher grade or stage) of the presenile dementia in the subject.
8. The application according to Embodiment 1, wherein the present application provides a product (e.g., kit or system) for diagnosis of presenile dementia and mild cognitive impairment, and/or for evaluation of progression (e.g., a grade or stage) of the presenile dementia, comprising:
   (i) a substance for detecting a DNA damage-inducible transcript 4-like (DDIT4L) intron retention spliced product (DIR) from a sample of a subject; and
   (ii) optionally, other substances for diagnosis of the presenile dementia and mild cognitive impairment, and/or for evaluation of progression (e.g., a grade or stage) of the presenile dementia, for example, a substance for detecting an existing marker for the presenile dementia,
   for example, in some cases:
   (i) the substance in (i) is selected from one or more of the group consisting of:
      a reagent, an apparatus or their combination for detecting the DIR;
      a substance for detecting the DIR at the gene level and/or protein level, such as a substance for use in one or more detection techniques or methods selected from the group consisting of:
         immunohistochemistry (e.g., immunofluorescence analysis, chemiluminescence, reverse enzyme-linked immunosorbent assay, an immunocolloidal gold method), Western blotting, Northern blotting, PCR, and a biochip method; or
         a substance specific to the DIR, for example, an antibody (preferably, a monoclonal antibody) of the substance; a probe, gene chip, PCR primer, or gRNA specific to the spliced product; and/or
   (ii) other substances in (ii) are selected from: a substance for detecting one or more other markers for presenile dementia, for example: AD7C-NTP, pTau-181, pTau-217, and Aβ1-42; and/or, a reagent for imaging of brain amyloid protein; and/or, a reagent for diagnosis of the imaging of the subject's brain.
9. A system for diagnosis of presenile dementia and mild cognitive impairment, and/or for evaluation of progression (e.g., a grade or stage) of the presenile dementia, comprising the following devices:
   (a) a device for collecting and/or receiving data on a DIR level in a DNA damage-inducible transcript 4-like (DDIT4L) intron retention spliced product (DIR) from a sample; and
   (b) a device for analyzing the data to diagnose presenile dementia and mild cognitive impairment, and/or evaluate the progression (e.g., a grade or stage) of the presenile dementia,
   wherein an increase in the DIR level in the sample compared to the normal control value indicates that the subject suffers from the presenile dementia or mild cognitive impairment; or, an increase in the DIR level in the sample compared to a mild cognitive impairment control or an early control level in a subject who has been diagnosed with presenile dementia indicates some development (e.g., a higher grade or stage) of the presenile dementia in the subject.
10. A method for screening of a medicament for treatment of presenile dementia, comprising: detecting an effect of a candidate medicament on the level of a DNA damage-inducible transcript 4-like (DDIT4L) intron retention spliced product (DIR) in a subject, wherein after the use of the candidate medicament, a decreased level indicates that the candidate medicament has a therapeutic effect on the presenile dementia.

Not wishing to be bound by any particular theory, the following examples are merely to set forth the detection method, application or the like of the present application, and are not intended to limit the scope of the invention of the present application.

### Examples

The present application is further set forth below in conjunction with specific examples. It should be understood that these examples are intended only to explain the present application, instead of limiting the scope of the present application. Those skilled in the art can make appropriate modifications and changes to the present application, and these modifications and changes shall fall within the scope of the present application.

For the experimental methods without specifying specific conditions in the following examples, the conventional methods in the art can be used (for example, see "Molecular Cloning: A Laboratory Manual", Third Edition, New York: Cold Spring Harbor Laboratory Press, 1989), or as recommended by the supplier. DNA sequencing methods are conventional methods in the art, or tests can also be provided by commercial companies.

Unless otherwise stated, percentages and parts are by weight. Unless otherwise defined, all technical and scientific terms used herein have the same meaning as those familiar to those skilled in the art. In addition, any methods and materials that are similar or equivalent to those described can be applied to the method of the present application. The preferred implementation methods and materials described in herein are for demonstration purposes only.

### Example 1 DIR Protein as Secreted Protein

### Test Materials and Methods

Method for plasmid construction and cell transfection: The cDNAs of human DIR were cloned into pCMV-flag vectors to express flag-DIR proteins. HEK293 cells (Institute of Biochemistry and Cell Biology, Chinese Academy of Sciences) were transfected with pCMV-flag plasmids and pCMV-flag-DIR plasmids using a Lippo3000 reagent; after being cultured for 48 hours, the cells and medium were harvested, and an appropriate amount of lysate was added; the resulting mixture was rotated for 30 min at 4°C for lysis, and centrifuged for 30 min at 12,000 g to obtain the supernatant; and a small amount of lysate was taken for Western Blot analysis.

Western Blot experiment: It was completed following the steps of SDS-PAGE electrophoresis, membrane transfer, immunoreaction and chemiluminescence (the antibodies used were purchased from GL Biochem (Shanghai) Ltd.).

### Results and Discussions

After the HEK293 cells were cultured for 48 hours, a large amount of DIR was expressed in the cells transfected with the pCMV-flag-DIR plasmids, and a large amount of DIR was also detected in the medium for the cells (FIG.1B). This indicates that DIR can be secreted into the extracellular medium through the intracellular secretion pathway after its transcription and translation into proteins. In terms of the amount of secretion, part of the intracellularly synthesized DIR is secreted into the extracellular medium, suggesting that DIR may also be secreted to outside of cells in a human body and then enter the blood circulation system. This provides a theoretical basis for taking DIR as a blood marker under some pathological conditions.

### Example 2 Changes in Content of DIR Proteins in Blood of AD Patients

### Test Materials and Methods

Tissue samples: Venous blood samples (58 samples in total) from AD and MCI patients, non-AD patients (patients with hypertension, coronary heart disease or diabetes) and normal people were sourced from Huashan Hospital Affiliated to Fudan University and Xuhui Central Hospital.

Methods for collecting venous blood and obtaining plasma: The blood was collected from the vein in cubital fossa of a patient into an EDTA vacuum tube by using the vacuum blood collection technique, and the tube was immediately inverted gently 8 times to fully mix the blood and an anticoagulant. Then, the tube was transported to the laboratory on ice, centrifuged for 10 min at 1500g at 4°C, and the supernatant was aspirated, aliquoted, and stored at -80°C.

Western Blot method for plasma: The aliquoted plasma was thawed on ice and diluted appropriately with PBS, followed by the addition of the buffer for denaturation for 5 minutes in a 100°C metal bath. The subsequent steps were the same as the Western Blot method for tissues.

ELISA method for plasma: The plasma sample was diluted at 1:1 with 1× diluent (OBT1998G, purchased from Bio-Rad); then, 100 µl of a blocking solution was added using a pipette for blockage for 1 h at 37°C; then, 100 µl of a proportionally diluted standard and diluted plasma were added into plate wells coated with capture antibodies (GL Biochem, AB009458) by using the pipette, the tip of which was changed for each sampling; the resulting mixture was incubated for 1.5 h at 37°C; the liquid in each well was removed, and the well was washed 3 times with 100 µl 1× washing solution (BUF031C, purchased from Bio-Rad); 100 µl of detection antibody (i. e., the DIR antibody IR-II-6, of which a VH amino acid sequence was as set forth in SEQ ID NO. 58, and of which a VL amino acid sequence was as set forth in SEQ ID NO. 60) was added to each well and incubated for 1 h at 37°C; the liquid in each well was removed, and the well was washed 3 times with 100 µl 1× washing solution; 100 µl of 1× avidin-HRP solution was added to each well, which was incubated for 1 h at 37°C; the liquid in each well was removed, and the well was washed 3 times with 100 µl 1× washing solution; 100 µl of 1× substrate reaction solution (BUF062C, purchased from Bio-Rad) was added to each well, which was incubated for 10-30 min at room temperature in the dark; and a microplate reader was used for detection at a wavelength of 655 nm, and a standard curve was established.

### Results and Discussions

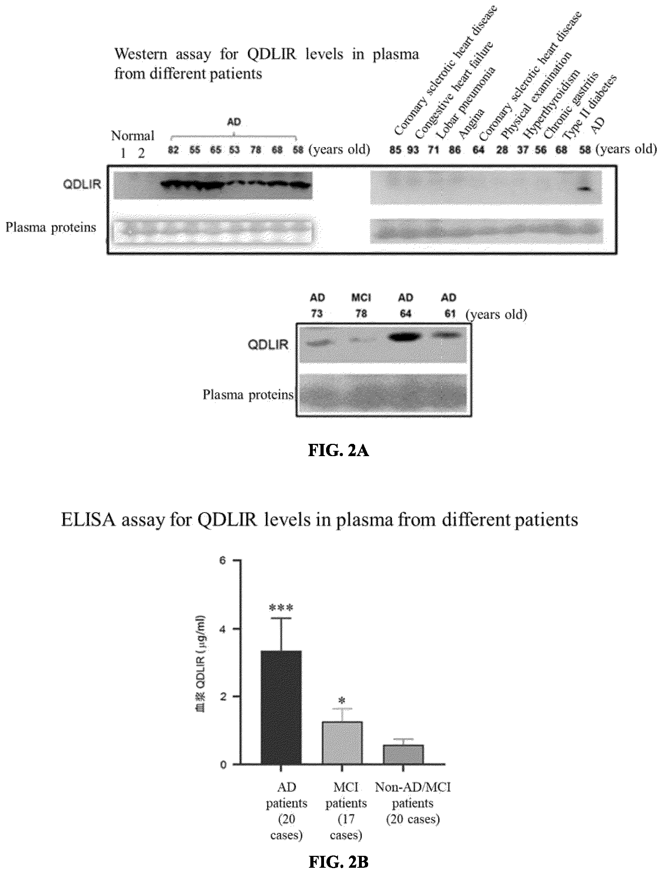

Based on the testing of different patients by clinical collection of blood samples from AD and MCI patients, non-AD patients and normal people, it was found that the content of DIR in the blood samples from the AD and MCI patients was the highest, and it was detectable in about 95% of patients; the blood samples from the MCI patients also contained a relatively high amount of DIR, and it was detectable in about 90% of patients; and in the blood samples from patients without cognitive impairment, there was no or very small amount of DIR detected, and no DIR was detected in the blood samples from the normal people (FIG.2A).

Through the ELISA method, it was found that the content of DIR in the blood samples from the AD patients was relatively high, reaching a level of 10-100 ug/ml, while the content of DIR in the non-AD patients was at the pg/ml level and was undetected in the normal people. Therefore, the DIR level in the AD patient was 103-104 times higher than that of other patients. This suggests very high specificity of DIR (FIG.2A and FIG.2B).

The above results indicate that DIR has a high concentration in the blood of the AD and MCI patients and can be used as a blood marker to detect the presence of AD, in particular for patients with mild cognitive impairment.

Moreover, the content of DIR is related to the severity of AD (see FIG.2B).

### Example 3 Methodological Study on DIR Protein Detection

The DIR protein (GL Biochem, 752084) was prepared into a 1 mg/ml stock solution with a DPBS solution, which was diluted with an ELISA washing solution (BUF031C, purchased from Bio-Rad) before the experiment to prepare a standard DIR protein solution.

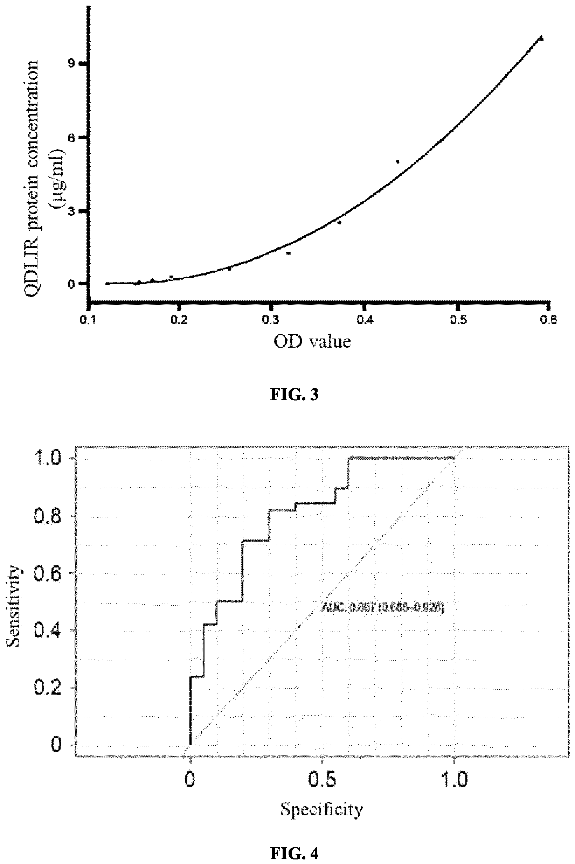

The concentration of standard DIR protein was detected by ELISA, and polynomial regression was used for fitting. The resulting standard curve was shown in FIG. 3.

The R2 value of this regression model was 0.99, indicating a high degree of fitting; the shaded part showed the 95% confidence interval of the regression model; and as can be seen from the figure, the confidence interval was narrow, indicating high confidence level of the DIR content in the sample as estimated based on this model.

### Example 4 R°C Curve with DIR as Clinical Detection Indicator

The blood samples clinically collected from AD and MCI patients, non-AD patients and normal people (same as Example 2) were detected by ELISA; and based on the detection results, a logistic regression model was established from the achieved DIR concentration using the "pROC" package analysis of R software (Version 3.6.2); and the self-service sampling method was used for analysis to obtain the ROC curve.

The results were shown in FIG. 4. The results show that good prediction results can be achieved by modeling using the single parameter of DIR only, with AUC=0.807 (95% confidence interval: 0.688-0.926).

### Example 5 Changes in Content of DIR Protein in Blood of Patients with Different Types of MCI

### Test Materials and Methods

Tissue samples: Venous blood samples from patients with different types of MCI and normal follow-up people were sourced from Huashan Hospital Affiliated to Fudan University.

Methods for collecting venous blood and obtaining plasma: Same as Example 2.

ELISA method for plasma: Same as Example 2.

MCI classification method: aMCI-s: single-domain amnestic MCI; aMCI-m: multi-domain amnestic MCI; naMCI-s: single-domain non-amnestic MCI; and naMCI-m: multi-domain non-amnestic MCI.

In Example 5, there were 122 samples from aMCI-m type patients; 85 samples from aMCI-s type patients; 11 samples from naMCI-m type patients; 88 samples from naMCI-s type patients; and 279 samples from normal follow-up people.

### Results and Discussions

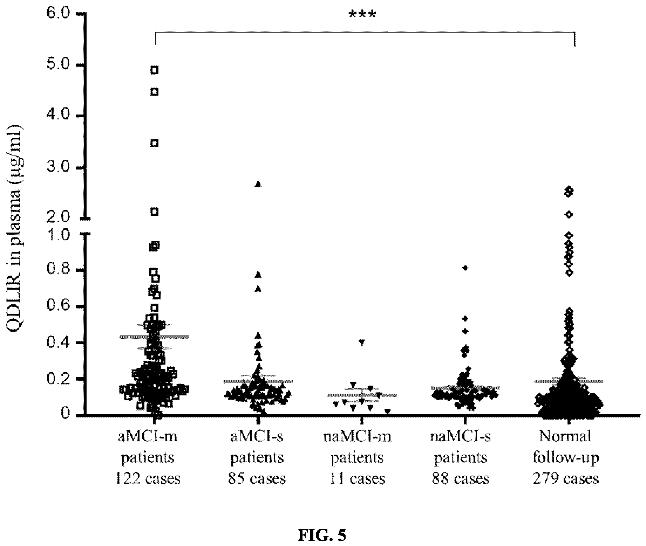

Based on the detection of the blood samples clinically collected from patients with different types of MCI and normal follow-up people, it was found that the content of DIR in the blood samples of MCI patients with aMCI-m type was increased, exhibiting a significant difference compared with the normal follow-up people (FIG. 5).

Through the ELISA method, it was found that the content of DIR in the blood of aMCI-m type patients was high, and the aMCI-m type patients were more likely to progress to AD than patients with other types of MCI, suggesting that DIR was an indicator capable of distinguishing aMCI-m types.

The above results indicate that DIR can be used as an effective diagnostic marker for AD.

### Example 6 Changes in Content of DIR Protein in Blood of Patients with Different Types of MCI

### Test Materials and Methods

Tissue samples: Venous blood samples from patients with different types of MCI, AD patients and normal follow-up people were sourced from Huashan Hospital Affiliated to Fudan University.

Methods for collecting venous blood and obtaining plasma: Same as Example 2.

ELISA method for plasma: The method for DIR was the same as that in Example 2; and Tau, Aβ40 and Aβ42 were subjected to commission detection by the triple kit (Product No.: 101995) of Hangzhou G-Bio Co., Ltd. according to its operating procedures.

MCI classification method: Same as Example 5.

### Results and Discussions

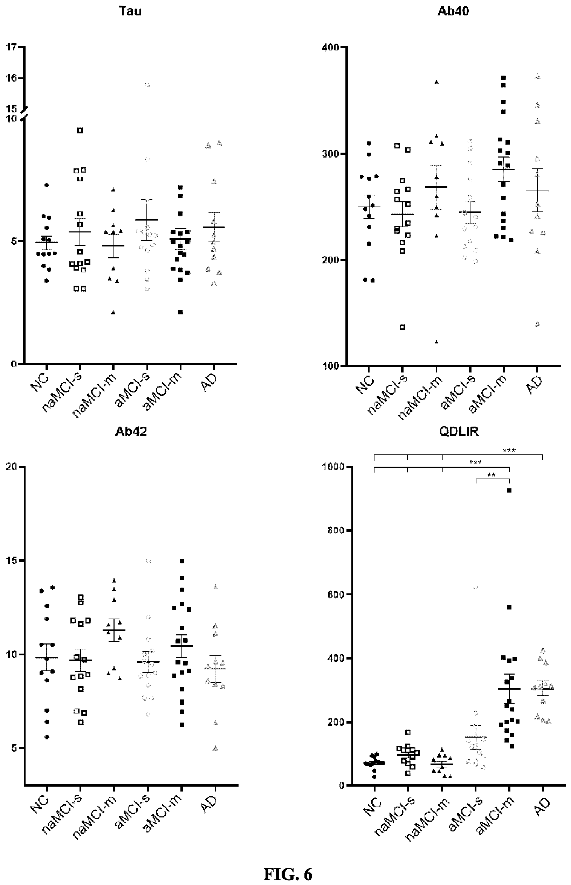

Based on our detection of the blood samples clinically collected from patients with different types of MCI, AD patients and normal follow-up people, it was found that the content of DIR in the blood samples from aMCI-m type patients and AD patients was increased, exhibiting a significant difference compared with the normal follow-up people and the patients with other MCI types; and the existing detection of indicators such as Tau, Aβ40 and Aβ42 in blood samples showed no significant different between the groups, although some indicators (such as Aβ40) showed the tendency to increase in the blood from aMCI-m type patients and AD patients (see FIG. 6).

Through the ELISA method, it was found that the content of DIR in the blood from aMCI-m type patients and AD patients was higher. It can be seen that compared with the existing auxiliary diagnostic indicators Tau, Aβ40 and Aβ42, DIR can more clearly distinguish the aMCI-m type patients, the AD patients and other types of MCI patients or normal people in the early stage. Therefore, DIR can be used as an effective diagnostic marker for aMCI-m and AD.

### Example 7 Changes in Content of DIR Proteins in Blood and Cerebrospinal Fluid from Patients

### Test Materials and Methods

Tissue samples: Venous blood samples and cerebrospinal fluid samples from the same patient (one case for each of cerebral edema, meningioma and glioma) were both sourced from Huashan Hospital Affiliated to Fudan University.

Methods for collecting venous blood and obtaining plasma: Same as Example 2.

Cerebrospinal fluid collection method: The cerebrospinal fluid was obtained from the patient during surgery.

ELISA method for plasma and cerebrospinal fluid: Same as Example 2.

### Results and Discussions

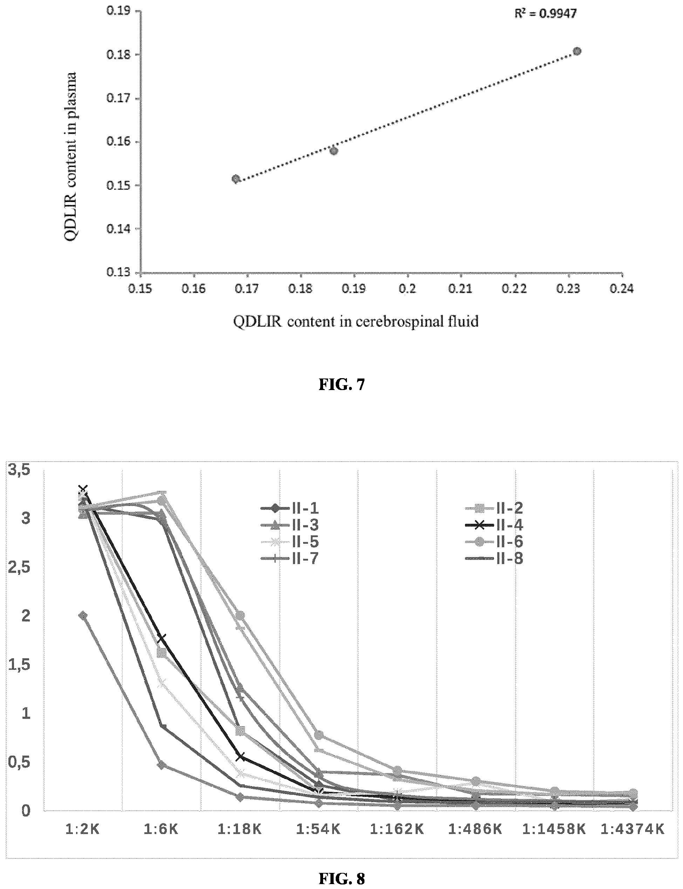

The content of DIR in the plasma and in the cerebrospinal fluid was highly consistent, with an R2 value of 0. 99 (see FIG. 7).

Through the ELISA method, the content of DIR in the blood from the patient was consistent with that in the cerebrospinal fluid, indicating that the content detected in the blood could directly reflect the content of DIR in the cerebrospinal fluid, suggesting that the DIR described in the present application is also suitable for use as an effective diagnostic marker in the case of taking the cerebrospinal fluid as a sample.

### Example 8 Detection of Binding Activity of DIR Antibodies

The sequencing results of DIR antibodies IR-II-1 to IR-II-10 (IR-II-10 was A2D10) were shown in Table 1 below.

**Table 1**

| | HCDR1 | HCDR2 | HCDR3 | LCDR1 | LCDR2 | LCDR3 | VH | VL |
|---|---|---|---|---|---|---|---|---|
| IR-II-1 | SEQ ID NO. 20 | SEQ ID NO. 21 | SEQ ID NO.22 | SEQ ID NO. 25 | SEQ ID NO. 26 | SEQ ID NO. 27 | SEQ ID NO. 23 | SEQ ID NO. 28 |
| IR-II-2 | SEQ ID NO. 20 | SEQ ID NO. 21 | SEQ ID NO. 30 | SEQ ID NO. 25 | SEQ ID NO. 26 | SEQ ID NO. 27 | SEQ ID NO. 31 | SEQ ID NO. 33 |
| IR-II-3 | SEQ ID NO. 35 | SEQ ID NO. 36 | SEQ ID NO. 37 | SEQ ID NO. 25 | SEQ ID NO. 26 | SEQ ID NO. 27 | SEQ ID NO. 38 | SEQ ID NO. 40 |
| IR-II-4 | SEQ ID NO. 20 | SEQ ID NO. 42 | SEQ ID NO. 43 | SEQ ID NO. 25 | SEQ ID NO. 26 | SEQ ID NO. 27 | SEQ ID NO. 44 | SEQ ID NO. 46 |
| IR-II-5 | SEQ ID NO. 20 | SEQ ID NO. 48 | SEQ ID NO. 49 | SEQ ID NO. 52 | SEQ ID NO. 26 | SEQ ID NO. 53 | SEQ ID NO. 50 | SEQ ID NO. 54 |
| IR-II-6 | SEQ ID NO. 56 | SEQ ID NO. 36 | SEQ ID NO. 57 | SEQ ID NO. 25 | SEQ ID NO. 26 | SEQ ID NO. 27 | SEQ ID NO. 58 | SEQ ID NO. 60 |
| IR-II-7 | SEQ ID NO. 35 | SEQ ID NO. 36 | SEQ ID NO. 62 | SEQ ID NO. 25 | SEQ ID NO. 26 | SEQ ID NO. 27 | SEQ ID NO. 63 | SEQ ID NO. 60 |
| IR-II-8 | SEQ ID NO. 35 | SEQ ID NO. 42 | SEQ ID NO. 37 | SEQ ID NO. 52 | SEQ ID NO. 26 | SEQ ID NO. 53 | SEQ ID NO. 65 | SEQ ID NO. 54 |
| IR-II-9 | SEQ ID NO. 56 | SEQ ID NO. 36 | SEQ ID NO. 57 | SEQ ID NO. 52 | SEQ ID NO. 26 | SEQ ID NO. 67 | SEQ ID NO. 58 | SEQ ID NO. 68 |
| A2D10 | SEQ ID NO. 10 | SEQ ID NO. 11 | SEQ ID NO. 12 | SEQ ID NO. 15 | SEQ ID NO. 16 | SEQ ID NO. 17 | SEQ ID NO. 13 | SEQ ID NO. 18 |

The binding activity of the above antibodies IR-II-1 to IR-II-10 to fragments DIR-II was detected. The method specifically comprises:

The target fragment DIR-II was coated on a 96-well plate using a coating buffer and blocked with a blocking buffer. Next, the monoclonal antibodies (1 mg/mL) were diluted in equal proportions (1:2k, 1:6k, 1:18k, 1:54k, 1:162k, 1:486k, 1:1458k, 1:4374k), added to single wells using a pipette, shaken evenly for 2 h at 37°C, and washed three times with a washing buffer. Then, a secondary antibody with horseradish peroxidase (1:5k) was added and incubated at 37°C. After washing three times, an appropriate volume of TMB substrate solution was added, and the resulting mixture was incubated at 37°C for a certain period of time, and then placed under a microplate reader to record the absorbance value of the sample at a wavelength of 450 nm.

The results were shown in Table 2 and FIG. 8. From these results, it can be seen that the above antibodies IR-II-1 to IR-II-10 specifically bind to the fragments DIR-II at a working concentration of about 10 ng/ml or above. Therefore, all the above antibodies IR-II-1 to IR-II-10 can be used for the detection of DIR proteins and the diagnosis of aMCI-m and AD.

**Table 2**

| Valence | IR-II-1 | IR-II-2 | IR-II-3 | IR-II-4 | IR-II-5 | IR-II-6 | IR-II-7 | IR-II-8 | IR-II-9 | IR-II-10 (A2D10) |
|---|---|---|---|---|---|---|---|---|---|---|
| 1:2k | 3.1394 | 3.229 | 3.0488 | 3.2953 | 3.2435 | 3.0937 | 3094 | 3.1999 | 3.112 | 2.0055 |
| 1:6k | 2.9827 | 1.6224 | 3.052 | 1.7675 | 1.313 | 3.1805 | 2.9775 | 0.8698 | 3.2696 | 0.4725 |
| 1:18k | 0.8184 | 0.823 | 1.2676 | 0.5554 | 0.3867 | 2.0055 | 1.1649 | 0.2571 | 1.8719 | 0.1422 |
| 1:54k | 0.2637 | 0.193 | 0.3995 | 0.1857 | 0.1585 | 0.7798 | 0.3497 | 0.138 | 0.6219 | 0.0783 |
| 1:162k | 0.1092 | 0.1398 | 0.3663 | 0.1361 | 0.1898 | 0.4141 | 0.1641 | 0.0911 | 0.3182 | 0.0517 |
| 1:486k | 0.0656 | 0.1116 | 0.1734 | 0.0955 | 0.2848 | 0.3057 | 0.1207 | 0.0864 | 0.2072 | 0.0517 |
| 1:1458k | 0.0653 | 0.0839 | 0.1657 | 0.0751 | 0.0911 | 0.2004 | 0.1034 | 0.0906 | 0.1692 | 0.0501 |
| 1:4374k | 0.0934 | 0.1149 | 0.1532 | 0.0775 | 0.0868 | 0.18 | 0.0867 | 0.0887 | 0.1931 | 0.0427 |

All the documents mentioned in the present application are hereby incorporated by reference in their entireties, just as each document is cited separately as a reference. In addition, it should be understood that various modifications or changes can be made by those skilled in the art after reading the above teachings of the present application, and these equivalent forms shall also fall within the scope defined by the claims appended hereto.

## Claims

1. Use of a DNA damage-inducible transcript 4-like DDIT4L intron retention spliced product DIR, and/or a nucleic acid encoding said DIR, in diagnosis of a disease and/or in evaluation of progression of said disease, wherein said disease comprises cognitive impairment.

2. Use of a DNA damage-inducible transcript 4-like DDIT4L intron retention spliced product DIR, and/or a nucleic acid encoding said DIR, in diagnosis of a disease and/or in evaluation of progression of said disease, wherein said disease comprises a neurodegenerative disease.

3. The use according to any one of claims 1 to 2, wherein said DIR comprises the amino acid sequence as set forth in SEQ ID NO: 1.

4. The use according to any one of claims 1 to 3, wherein said nucleic acid encoding said DIR comprises a nucleotide sequence as set forth in SEQ ID NO: 8.

5. The use according to any one of claims 1 to 4, wherein said nucleic acid encoding said DIR comprises a nucleotide sequence as set forth in SEQ ID NO: 93.

6. The use according to any one of claims 1 to 5, wherein said cognitive impairment comprises cognitive impairment caused by normal aging, Lewy body dementia LBD, frontotemporal dementia and/or vascular dementia.

7. The use according to any one of claims 1 to 6, wherein the cognitive impairment-inducing diseases comprise Alzheimer's disease, multi-infarct type, Parkinson's disease, AIDS and/or Creutzfeldt-Jakob disease CJD.

8. The use according to any one of claims 1 to 7, wherein said cognitive impairment comprises mild cognitive impairment MCI, intermediate cognitive impairment and late cognitive impairment.

9. The use according to any one of claims 1 to 8, wherein said cognitive impairment comprises multi-domain amnestic MCI aMCI-m.

10. The use according to any one of claims 2 to 9, wherein said neurodegenerative disease comprises an acute neurodegenerative disease and a chronic neurodegenerative disease.

11. The use according to any one of claims 2 to 10, wherein said neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

12. The use according to any one of claims 2 to 11, wherein said neurodegenerative disease comprises Alzheimer's disease, Parkinson's disease, multiple sclerosis MS, amyotrophic lateral sclerosis ALS and/or Huntington's disease HD.

13. The use according to any one of claims 2 to 12, wherein said neurodegenerative disease comprises presenile dementia.

14. The use according to any one of claims 2 to 13, wherein said neurodegenerative disease comprises early presenile dementia, intermediate presenile dementia and/or late presenile dementia.

15. Use of a substance for detecting a DNA damage-inducible transcript 4-like DDIT4L intron retention spliced product DIR, and/or a nucleic acid encoding said DIR, in preparation of a product for diagnosis of a disease and/or in evaluation of progression of said disease, wherein said disease comprises cognitive impairment.

16. Use of a substance for detecting a DNA damage-inducible transcript 4-like DDIT4L intron retention spliced product DIR, and/or a nucleic acid encoding said DIR, in preparation of a product for diagnosis of a disease and/or in evaluation of progression of said disease, wherein said disease comprises a neurodegenerative disease.

17. The use according to any one of claims 15 to 16, wherein said DIR comprises the amino acid sequence as set forth in SEQ ID NO: 1.

18. The use according to any one of claims 15 to 17, wherein said nucleic acid encoding said DIR comprises a nucleotide sequence as set forth in SEQ ID NO: 8.

19. The use according to any one of claims 15 to 18, wherein said nucleic acid encoding said DIR comprises a nucleotide sequence as set forth in SEQ ID NO: 93.

20. The use according to any one of claims 15 to 19, wherein said cognitive impairment comprises cognitive impairment caused by normal aging, Lewy body dementia LBD, frontotemporal dementia and/or vascular dementia.

21. The use according to any one of claims 15 to 20, wherein the cognitive impairment-inducing diseases comprise Alzheimer's disease, multi-infarct type, Parkinson's disease, AIDS and/or Creutzfeldt-Jakob disease CJD.

22. The use according to any one of claims 15 to 21, wherein said cognitive impairment comprises mild cognitive impairment MCI, intermediate cognitive impairment and late cognitive impairment.

23. The use according to any one of claims 15 to 22, wherein said cognitive impairment comprises multi-domain amnestic MCI aMCI-m.

24. The use according to any one of claims 16 to 23, wherein said neurodegenerative disease comprises an acute neurodegenerative disease and a chronic neurodegenerative disease.

25. The use according to any one of claims 16 to 24, wherein said neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

26. The use according to any one of claims 16 to 25, wherein said neurodegenerative disease comprises Alzheimer's disease, Parkinson's disease, multiple sclerosis MS, amyotrophic lateral sclerosis ALS and/or Huntington's disease HD.

27. The use according to any one of claims 16 to 26, wherein said neurodegenerative disease comprises presenile dementia.

28. The use according to any one of claims 16 to 27, wherein said neurodegenerative disease comprises early presenile dementia, intermediate presenile dementia and/or late presenile dementia.

29. The use according to any one of claims 15 to 28, wherein said substance comprises a reagent and/or an apparatus capable of detecting said DIR, and/or said nucleic acid encoding said DIR.

30. The use according to any one of claims 15 to 29, wherein said substance comprises a reagent for specifically detecting said DIR, and/or said nucleic acid encoding said DIR.

31. The use according to any one of claims 15 to 30, wherein said substance comprises an antigen-binding protein capable of specifically binding to said DIR, a probe capable of specifically binding to said DIR, a primer capable of specifically amplifying said nucleic acid encoding said DIR, a gene chip capable of specifically detecting said nucleic acid encoding said DIR, an aptamer capable of specifically binding to said nucleic acid encoding said DIR, and/or a guide RNA capable of specifically binding to said nucleic acid encoding said DIR.

32. The use according to claim 31, wherein said antigen-binding protein comprises LCDR2, which comprises the amino acid sequence as set forth in SEQ ID NO: 74.

33. The use according to claim 32, wherein said LCDR2 comprises the amino acid sequence as set forth in SEQ ID NO: 26 or 16.

34. The use according to any one of claims 31 to 33, wherein said antigen-binding protein comprises LCDR1, which comprises the amino acid sequence as set forth in SEQ ID NO: 73.

35. The use according to claim 34, wherein said LCDR1 comprises the amino acid sequence as set forth in any one of SEQ ID NOs: 25, 52 and 15.

36. The use according to any one of claims 31 to 35, wherein said antigen-binding protein comprises LCDR3, which comprises the amino acid sequence as set forth in SEQ ID NO: 75.

37. The use according to claim 36, wherein said LCDR3 comprises the amino acid sequence as set forth in any one of SEQ ID NOs: 27, 53, 67 and 17.

38. The use according to any one of claims 31 to 37, wherein said antigen-binding protein comprises LCDR1, LCDR2 and LCDR3, wherein
a) said LCDR1 comprises the amino acid sequence as set forth in SEQ ID NO: 25, said LCDR2 comprises the amino acid sequence as set forth in SEQ ID NO: 26, and said LCDR3 comprises the amino acid sequence as set forth in SEQ ID NO: 27;
b) said LCDR1 comprises the amino acid sequence as set forth in SEQ ID NO: 52, said LCDR2 comprises the amino acid sequence as set forth in SEQ ID NO: 26, and said LCDR3 comprises the amino acid sequence as set forth in SEQ ID NO: 53;
c) said LCDR1 comprises the amino acid sequence as set forth in SEQ ID NO: 52, said LCDR2 comprises the amino acid sequence as set forth in SEQ ID NO: 26, and said LCDR3 comprises the amino acid sequence as set forth in SEQ ID NO: 67; or,
d) said LCDR1 comprises the amino acid sequence as set forth in SEQ ID NO: 15, said LCDR2 comprises the amino acid sequence as set forth in SEQ ID NO: 16, and said LCDR3 comprises the amino acid sequence as set forth in SEQ ID NO: 17.

39. The use according to any one of claims 31 to 38, wherein said antigen-binding protein comprises HCDR1, which comprises the amino acid sequence as set forth in SEQ ID NO: 70.

40. The use according to claim 39, wherein said HCDR1 comprises the amino acid sequence as set forth in any one of SEQ ID NOs: 20, 35, 56 and 10.

41. The use according to any one of claims 31 to 40, wherein said antigen-binding protein comprises HCDR2, which comprises the amino acid sequence as set forth in SEQ ID NO: 71.

42. The use according to claim 41, wherein said HCDR2 comprises the amino acid sequence as set forth in any one of SEQ ID NOs: 21, 36, 42, 48 and 11.

43. The use according to any one of claims 31 to 42, wherein said antigen-binding protein comprises HCDR3, which comprises the amino acid sequence as set forth in SEQ ID NO: 70.

44. The use according to claim 43, wherein said HCDR3 comprises the amino acid sequence as set forth in any one of SEQ ID NOs: 22, 30, 37, 43, 49, 57, 62 and 12.

45. The use according to any one of claims 31 to 44, wherein said antigen-binding protein comprises HCDR1, HCDR2 and HCDR3, wherein
a) said HCDR1 comprises the amino acid sequence as set forth in SEQ ID NO: 20, said HCDR2 comprises the amino acid sequence as set forth in SEQ ID NO: 21, and said HCDR3 comprises the amino acid sequence as set forth in SEQ ID NO: 22;
b) said HCDR1 comprises the amino acid sequence as set forth in SEQ ID NO: 20, said HCDR2 comprises the amino acid sequence as set forth in SEQ ID NO: 21, and said HCDR3 comprises the amino acid sequence as set forth in SEQ ID NO: 30;
c) said HCDR1 comprises the amino acid sequence as set forth in SEQ ID NO: 35, said HCDR2 comprises the amino acid sequence as set forth in SEQ ID NO: 36, and said HCDR3 comprises the amino acid sequence as set forth in SEQ ID NO: 37;
d) said HCDR1 comprises the amino acid sequence as set forth in SEQ ID NO: 20, said HCDR2 comprises the amino acid sequence as set forth in SEQ ID NO: 42, and said HCDR3 comprises the amino acid sequence as set forth in SEQ ID NO: 43;
e) said HCDR1 comprises the amino acid sequence as set forth in SEQ ID NO: 20, said HCDR2 comprises the amino acid sequence as set forth in SEQ ID NO: 48, and said HCDR3 comprises the amino acid sequence as set forth in SEQ ID NO: 49;
f) said HCDR1 comprises the amino acid sequence as set forth in SEQ ID NO: 56, said HCDR2 comprises the amino acid sequence as set forth in SEQ ID NO: 36, and said HCDR3 comprises the amino acid sequence as set forth in SEQ ID NO: 57;
g) said HCDR1 comprises the amino acid sequence as set forth in SEQ ID NO: 35, said HCDR2 comprises the amino acid sequence as set forth in SEQ ID NO: 36, and said HCDR3 comprises the amino acid sequence as set forth in SEQ ID NO: 62;
h) said HCDR1 comprises the amino acid sequence as set forth in SEQ ID NO: 35, said HCDR2 comprises the amino acid sequence as set forth in SEQ ID NO: 42, and said HCDR3 comprises the amino acid sequence as set forth in SEQ ID NO: 37;
i) said HCDR1 comprises the amino acid sequence as set forth in SEQ ID NO: 56, said HCDR2 comprises the amino acid sequence as set forth in SEQ ID NO: 36, and said HCDR3 comprises the amino acid sequence as set forth in SEQ ID NO: 57; or
j) said HCDR1 comprises the amino acid sequence as set forth in SEQ ID NO: 10, said HCDR2 comprises the amino acid sequence as set forth in SEQ ID NO: 11, and said HCDR3 comprises the amino acid sequence as set forth in SEQ ID NO: 12.

46. The use according to any one of claims 31 to 45, wherein said antigen-binding protein comprises a heavy chain variable region VH, which comprises the amino acid sequence as set forth in any one of SEQ ID NOs: 13, 23, 31, 38, 44, 50, 58, 63 and 65.

47. The use according to any one of claims 31 to 46, wherein said antigen-binding protein comprises a light chain variable region VL, which comprises the amino acid sequence as set forth in any one of SEQ ID NOs: 18, 28, 33, 40, 46, 54, 60 and 68.

48. The use according to any one of claims 31 to 47, wherein said antigen-binding protein comprises a heavy chain variable region VH and a light chain variable region VL, wherein:
a) said VH comprises the amino acid sequence as set forth in SEQ ID NO: 23 and said VL comprises the amino acid sequence as set forth in SEQ ID NO: 28;
b) said VH comprises the amino acid sequence as set forth in SEQ ID NO: 31 and said VL comprises the amino acid sequence as set forth in SEQ ID NO: 33;
c) said VH comprises the amino acid sequence as set forth in SEQ ID NO: 38 and said VL comprises the amino acid sequence as set forth in SEQ ID NO: 40;
d) said VH comprises the amino acid sequence as set forth in SEQ ID NO: 44 and said VL comprises the amino acid sequence as set forth in SEQ ID NO: 46;
e) said VH comprises the amino acid sequence as set forth in SEQ ID NO: 50 and said VL comprises the amino acid sequence as set forth in SEQ ID NO: 54;
f) said VH comprises the amino acid sequence as set forth in SEQ ID NO: 58 and said VL comprises the amino acid sequence as set forth in SEQ ID NO: 60;
g) said VH comprises the amino acid sequence as set forth in SEQ ID NO: 63 and said VL comprises the amino acid sequence as set forth in SEQ ID NO: 60;
h) said VH comprises the amino acid sequence as set forth in SEQ ID NO: 65 and said VL comprises the amino acid sequence as set forth in SEQ ID NO: 54;
i) said VH comprises the amino acid sequence as set forth in SEQ ID NO: 58 and said VL comprises the amino acid sequence as set forth in SEQ ID NO: 68; or
j) said VH comprises the amino acid sequence as set forth in SEQ ID NO: 13, and said VL comprises the amino acid sequence as set forth in SEQ ID NO: 18.

49. The use according to any one of claims 31 to 48, wherein said antigen-binding protein comprises a heavy chain constant region, which comprises an IgG-derived constant region.

50. The use according to claim 49, wherein said heavy chain constant region comprises a constant region derived from a protein selected from the group consisting of: IgG1, IgG2, IgG3 and IgG4.

51. The use according to any one of claims 31 to 50, wherein said antigen-binding protein comprises a light chain constant region, which comprises an Igx-derived constant region or an Igλ-derived constant region.

52. The use according to claim 51, wherein said light chain constant region comprises a human Igκ-derived constant region.

53. The use according to any one of claims 31 to 52, wherein said antigen-binding protein comprises an antibody or an antigen-binding fragment thereof.

54. The use according to claim 53, wherein said antigen-binding fragment is selected from the group consisting of: Fab, Fab', F(ab)2, a Fv fragment, F(ab')2, scFv, di-scFv, VHH and/or dAb.

55. The use according to any one of claims 53 to 54, wherein said antibody is selected from the group consisting of: a monoclonal antibody, a murine antibody and a chimeric antibody.

56. The use according to any one of claims 15 to 55, wherein said product comprises a diagnostic kit.

57. A diagnostic kit, comprising said substance of any one of claims 15 to 56, wherein said diagnostic kit is for use in diagnosis of cognitive impairment, and/or evaluation of progression of said cognitive impairment; and/or for use in diagnosis of a neurodegenerative disease, and/or evaluation of progression of said neurodegenerative disease.

58. The diagnostic kit according to claim 57, comprising an instruction manual recording a method of using said diagnostic kit for diagnosis of cognitive impairment, and/or evaluation of progression of said cognitive impairment; and/or for diagnosis of a neurodegenerative disease, and/or evaluation of progression of said neurodegenerative disease.

59. A method for diagnosing cognitive impairment, and/or evaluating progression of said cognitive impairment, comprising the step of: detecting, from a sample of a subject in need thereof, a content of a DNA damage-inducible transcript 4-like DDIT4L intron retention spliced product DIR, and/or of a nucleic acid encoding said DIR.

60. A method for diagnosing a neurodegenerative disease, and/or evaluating progression of said neurodegenerative disease, comprising the step of: detecting, from a sample of a subject in need thereof, a content of a DNA damage-inducible transcript 4-like DDIT4L intron retention spliced product DIR, and/or of a nucleic acid encoding said DIR.

61. The method according to any one of claims 59 to 60, wherein said DIR comprises the amino acid sequence as set forth in SEQ ID NO: 1.

62. The method according to any one of claims 59 to 61, wherein said nucleic acid encoding said DIR comprises a nucleotide sequence as set forth in SEQ ID NO: 8.

63. The method according to any one of claims 59 to 62, wherein said nucleic acid encoding said DIR comprises a nucleotide sequence as set forth in SEQ ID NO: 93.

64. The method according to any one of claims 59 to 63, wherein said cognitive impairment comprises cognitive impairment caused by normal aging, Lewy body dementia LBD, frontotemporal dementia and/or vascular dementia.

65. The method according to any one of claims 59 to 64, wherein the cognitive impairment-inducing diseases comprises Alzheimer's disease, multi-infarct type, Parkinson's disease, AIDS and/or Creutzfeldt-Jakob disease CJD.

66. The method according to any one of claims 59 to 65, wherein said cognitive impairment comprises mild cognitive impairment MCI, intermediate cognitive impairment and late cognitive impairment.

67. The method according to any one of claims 59 to 66, wherein said cognitive impairment comprises multi-domain amnestic MCI aMCI-m.

68. The method according to any one of claims 60 to 67, wherein said neurodegenerative disease comprises an acute neurodegenerative disease and a chronic neurodegenerative disease.

69. The method according to any one of claims 60 to 68, wherein said neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

70. The method according to any one of claims 60 to 69, wherein said neurodegenerative disease comprises Alzheimer's disease, Parkinson's disease, multiple sclerosis MS, amyotrophic lateral sclerosis ALS and/or Huntington's disease HD.

71. The method according to any one of claims 60 to 70, wherein said neurodegenerative disease comprises presenile dementia.

72. The method according to any one of claims 60 to 71, wherein said neurodegenerative disease comprises early presenile dementia, intermediate presenile dementia and/or late presenile dementia.

73. The method according to any one of claims 59 to 72, wherein said subject comprises a mammal.

74. The method according to any one of claims 59 to 73, wherein said subject comprises a tumor patient.

75. The method according to any one of claims 59 to 74, wherein said subject is in an old age stage.

76. The method according to any one of claims 59 to 75, wherein said sample comprises a blood sample and/or a tissue or cell sample.

77. The method according to any one of claims 59 to 76, wherein said sample comprises whole blood, serum, plasma, and/or cerebrospinal fluid.

78. The method according to any one of claims 59 to 77, wherein said method comprises: comparing the content of said DIR and/or of said nucleic acid encoding said DIR in the sample of said subject with a normal control value, wherein said normal control value comprises a content of said DIR and/or of said nucleic acid encoding said DIR in a normal subject.

79. The method according to claim 78, wherein said normal subject does not suffer from said cognitive impairment and/or said neurodegenerative disease.

80. The method according to any one of claims 78 to 79, wherein when the content of said DIR and/or of said nucleic acid encoding said DIR in the sample of said subject is significantly higher than said normal control value, said subject is diagnosed with said cognitive impairment and/or said neurodegenerative disease.

81. The method according to any one of claims 59 to 80, wherein said method comprises: comparing the content of said DIR and/or of said nucleic acid encoding said DIR in the sample of said subject with an early control value, wherein said early control value comprises a previously measured content of said DIR and/or of said nucleic acid encoding said DIR in said same subject.

82. The method according to claim 81, wherein said subject has been diagnosed with said cognitive impairment and/or said neurodegenerative disease.

83. The method according to any one of claims 81 to 82, wherein when the content of said DIR and/or of said nucleic acid encoding said DIR in the sample of said subject is significantly higher than said early control value, said subject is diagnosed with aggravated progression of said cognitive impairment and/or said neurodegenerative disease.

84. The method according to any one of claims 59 to 83, further comprising the step of detecting a content of a marker associated with said cognitive impairment and/or said neurodegenerative disease in a sample derived from said subject.

85. The method according to claim 84, wherein said marker associated with said cognitive impairment and/or said neurodegenerative disease comprises AD7C-NTP, pTau-181, pTau-217 and /or Aβ1-42.

86. The method according to any one of claims 59 to 85, further comprising the step of observing brain imaging of said subject.

87. A system for diagnosing cognitive impairment, and/or evaluating progression of said cognitive impairment, comprising a substance for detecting a DNA damage-inducible transcript 4-like DDIT4L intron retention spliced product DIR, and/or a nucleic acid encoding said DIR.

88. A system for diagnosing a neurodegenerative disease, and/or evaluating progression of said neurodegenerative disease, comprising a substance for detecting a DNA damage-inducible transcript 4-like DDIT4L intron retention spliced product DIR, and/or a nucleic acid encoding said DIR.

89. The system according to any one of claims 87 to 88, wherein said DIR comprises the amino acid sequence as set forth in SEQ ID NO: 1.

90. The system according to any one of claims 87 to 89, wherein said nucleic acid encoding said DIR comprises a nucleotide sequence as set forth in SEQ ID NO: 8.

91. The system according to any one of claims 87 to 90, wherein said nucleic acid encoding said DIR comprises a nucleotide sequence as set forth in SEQ ID NO: 93.

92. The system according to any one of claims 87 to 91, wherein said cognitive impairment comprises cognitive impairment caused by normal aging, Lewy body dementia LBD, frontotemporal dementia and/or vascular dementia.

93. The system according to any one of claims 87 to 92, wherein the cognitive impairment-inducing diseases comprises Alzheimer's disease, multi-infarct type, Parkinson's disease, AIDS and/or Creutzfeldt-Jakob disease CJD.

94. The system according to any one of claims 87 to 93, wherein said cognitive impairment comprises mild cognitive impairment MCI, intermediate MCI and late MCI.

95. The system according to any one of claims 87 to 94, wherein said cognitive impairment comprises multi-domain amnestic MCI aMCI-m.

96. The system according to any one of claims 88 to 95, wherein said neurodegenerative disease comprises an acute neurodegenerative disease and a chronic neurodegenerative disease.

97. The system according to any one of claims 88 to 96, wherein said neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

98. The system according to any one of claims 88 to 97, wherein said neurodegenerative disease comprises Alzheimer's disease, Parkinson's disease, multiple sclerosis MS, amyotrophic lateral sclerosis ALS and/or Huntington's disease HD.

99. The system according to any one of claims 88 to 98, wherein said neurodegenerative disease comprises presenile dementia.

100. The system according to any one of claims 88 to 99, wherein said neurodegenerative disease comprises early presenile dementia, intermediate presenile dementia and/or late presenile dementia.

101. The system according to any one of claims 87 to 100, which exists in a kit form.

102. The system according to any one of claims 87 to 101, comprising a collection module capable of collecting a sample from a subject in need thereof.

103. The system according to claim 102, wherein said subject comprises a mammal.

104. The system according to any one of claims 102 to 103, wherein said subject comprises a tumor patient.

105. The system according to any one of claims 102 to 104, wherein said subject is in an old age stage.

106. The system according to any one of claims 102 to 105, wherein said sample comprises a blood sample and/or a tissue or cell sample.

107. The system according to any one of claims 102 to 106, wherein said sample comprises whole blood, serum, plasma, and/or cerebrospinal fluid.

108. The system according to any one of claims 87 to 107, comprising a detection module capable of detecting a content value of said DIR and/or of said nucleic acid encoding said DIR.

109. The system according to claim 108, wherein said detection module detects a content value of said DIR and/or of said nucleic acid encoding said DIR in said sample acquired by said collection module.

110. The system according to any one of claims 108 to 109, wherein said detection module comprises a substance capable of detecting a content value of said DIR and/or of said nucleic acid encoding said DIR.

111. The system according to claim 110, wherein said substance comprises an antigen-binding protein capable of specifically binding to said DIR, a probe capable of specifically binding to said DIR, a primer capable of specifically amplifying said nucleic acid encoding said DIR, a gene chip capable of specifically detecting said nucleic acid encoding said DIR, an aptamer capable of specifically binding to said nucleic acid encoding said DIR, and/or a guide RNA capable of specifically binding to said nucleic acid encoding said DIR.

112. The system according to claim 111, wherein said antigen-binding protein comprises LCDR2, which comprises the amino acid sequence as set forth in SEQ ID NO: 74.

113. The system according to claim 112, wherein said LCDR2 comprises the amino acid sequence as set forth in SEQ ID NO: 26 or 16.

114. The system according to any one of claims 112 to 113, wherein said antigen-binding protein comprises LCDR1, which comprises the amino acid sequence as set forth in SEQ ID NO: 73.

115. The system according to claim 114, wherein said LCDR1 comprises the amino acid sequence as set forth in any one of SEQ ID NOs: 25, 52 and 15.

116. The system according to any one of claims 111 to 115, wherein said antigen-binding protein comprises LCDR3, which comprises the amino acid sequence as set forth in SEQ ID NO: 75.

117. The system according to claim 116, wherein said LCDR3 comprises the amino acid sequence as set forth in any one of SEQ ID NOs: 27, 53, 67 and 17.

118. The system according to any one of claims 111 to 117, wherein said antigen-binding protein comprises LCDR1, LCDR2 and LCDR3, wherein
a) said LCDR1 comprises the amino acid sequence as set forth in SEQ ID NO: 25, said LCDR2 comprises the amino acid sequence as set forth in SEQ ID NO: 26, and said LCDR3 comprises the amino acid sequence as set forth in SEQ ID NO: 27;
b) said LCDR1 comprises the amino acid sequence as set forth in SEQ ID NO: 52, said LCDR2 comprises the amino acid sequence as set forth in SEQ ID NO: 26, and said LCDR3 comprises the amino acid sequence as set forth in SEQ ID NO: 53;
c) said LCDR1 comprises the amino acid sequence as set forth in SEQ ID NO: 52, said LCDR2 comprises the amino acid sequence as set forth in SEQ ID NO: 26, and said LCDR3 comprises the amino acid sequence as set forth in SEQ ID NO: 67; or,
d) said LCDR1 comprises the amino acid sequence as set forth in SEQ ID NO: 15, said LCDR2 comprises the amino acid sequence as set forth in SEQ ID NO: 16, and said LCDR3 comprises the amino acid sequence as set forth in SEQ ID NO: 17.

119. The system according to any one of claims 111 to 118, wherein said antigen-binding protein comprises HCDR1, which comprises the amino acid sequence as set forth in SEQ ID NO: 70.

120. The system according to claim 119, wherein said HCDR1 comprises the amino acid sequence as set forth in any one of SEQ ID NOs: 20, 35, 56 and 10.

121. The system according to any one of claims 111 to 120, wherein said antigen-binding protein comprises HCDR2, which comprises the amino acid sequence as set forth in SEQ ID NO: 71.

122. The system according to claim 121, wherein said HCDR2 comprises the amino acid sequence as set forth in any one of SEQ ID NOs: 21, 36, 42, 48 and 11.

123. The system according to any one of claims 111 to 122, wherein said antigen-binding protein comprises HCDR3, which comprises the amino acid sequence as set forth in SEQ ID NO: 70.

124. The system according to claim 123, wherein said HCDR3 comprises the amino acid sequence as set forth in any one of SEQ ID NOs: 22, 30, 37, 43, 49, 57, 62 and 12.

125. The system according to any one of claims 111 to 124, wherein said antigen-binding protein comprises HCDR1, HCDR2 and HCDR3, wherein
a) said HCDR1 comprises the amino acid sequence as set forth in SEQ ID NO: 20, said HCDR2 comprises the amino acid sequence as set forth in SEQ ID NO: 21, and said HCDR3 comprises the amino acid sequence as set forth in SEQ ID NO: 22;
b) said HCDR1 comprises the amino acid sequence as set forth in SEQ ID NO: 20, said HCDR2 comprises the amino acid sequence as set forth in SEQ ID NO: 21, and said HCDR3 comprises the amino acid sequence as set forth in SEQ ID NO: 30;
c) said HCDR1 comprises the amino acid sequence as set forth in SEQ ID NO: 35, said HCDR2 comprises the amino acid sequence as set forth in SEQ ID NO: 36, and said HCDR3 comprises the amino acid sequence as set forth in SEQ ID NO: 37;
d) said HCDR1 comprises the amino acid sequence as set forth in SEQ ID NO: 20, said HCDR2 comprises the amino acid sequence as set forth in SEQ ID NO: 42, and said HCDR3 comprises the amino acid sequence as set forth in SEQ ID NO: 43;
e) said HCDR1 comprises the amino acid sequence as set forth in SEQ ID NO: 20, said HCDR2 comprises the amino acid sequence as set forth in SEQ ID NO: 48, and said HCDR3 comprises the amino acid sequence as set forth in SEQ ID NO: 49;
f) said HCDR1 comprises the amino acid sequence as set forth in SEQ ID NO: 56, said HCDR2 comprises the amino acid sequence as set forth in SEQ ID NO: 36, and said HCDR3 comprises the amino acid sequence as set forth in SEQ ID NO: 57;
g) said HCDR1 comprises the amino acid sequence as set forth in SEQ ID NO: 35, said HCDR2 comprises the amino acid sequence as set forth in SEQ ID NO: 36, and said HCDR3 comprises the amino acid sequence as set forth in SEQ ID NO: 62;
h) said HCDR1 comprises the amino acid sequence as set forth in SEQ ID NO: 35, said HCDR2 comprises the amino acid sequence as set forth in SEQ ID NO: 42, and said HCDR3 comprises the amino acid sequence as set forth in SEQ ID NO: 37;
i) said HCDR1 comprises the amino acid sequence as set forth in SEQ ID NO: 56, said HCDR2 comprises the amino acid sequence as set forth in SEQ ID NO: 36, and said HCDR3 comprises the amino acid sequence as set forth in SEQ ID NO: 57; or
j) said HCDR1 comprises the amino acid sequence as set forth in SEQ ID NO: 10, said HCDR2 comprises the amino acid sequence as set forth in SEQ ID NO: 11, and said HCDR3 comprises the amino acid sequence as set forth in SEQ ID NO: 12.

126. The system according to any one of claims 111 to 125, wherein said antigen-binding protein comprises a heavy chain variable region VH, which comprises the amino acid sequence as set forth in any one of SEQ ID NOs: 13, 23, 31, 38, 44, 50, 58, 63 and 65.

127. The system according to any one of claims 111 to 126, wherein said antigen-binding protein comprises a light chain variable region VL, which comprises the amino acid sequence as set forth in any one of SEQ ID NOs: 18, 28, 33, 40, 46, 54, 60 and 68.

128. The system according to any one of claims 111 to 127, wherein said antigen-binding protein comprises a heavy chain variable region VH and a light chain variable region VL, wherein:
a) said VH comprises the amino acid sequence as set forth in SEQ ID NO: 23 and said VL comprises the amino acid sequence as set forth in SEQ ID NO: 28;
b) said VH comprises the amino acid sequence as set forth in SEQ ID NO: 31 and said VL comprises the amino acid sequence as set forth in SEQ ID NO: 33;
c) said VH comprises the amino acid sequence as set forth in SEQ ID NO: 38 and said VL comprises the amino acid sequence as set forth in SEQ ID NO: 40;
d) said VH comprises the amino acid sequence as set forth in SEQ ID NO: 44 and said VL comprises the amino acid sequence as set forth in SEQ ID NO: 46;
e) said VH comprises the amino acid sequence as set forth in SEQ ID NO: 50 and said VL comprises the amino acid sequence as set forth in SEQ ID NO: 54;
f) said VH comprises the amino acid sequence as set forth in SEQ ID NO: 58 and said VL comprises the amino acid sequence as set forth in SEQ ID NO: 60;
g) said VH comprises the amino acid sequence as set forth in SEQ ID NO: 63 and said VL comprises the amino acid sequence as set forth in SEQ ID NO: 60;
h) said VH comprises the amino acid sequence as set forth in SEQ ID NO: 65 and said VL comprises the amino acid sequence as set forth in SEQ ID NO: 54;
i) said VH comprises the amino acid sequence as set forth in SEQ ID NO: 58 and said VL comprises the amino acid sequence as set forth in SEQ ID NO: 68; or
j) said VH comprises the amino acid sequence as set forth in SEQ ID NO: 13, and said VL comprises the amino acid sequence as set forth in SEQ ID NO: 18.

129. The system according to any one of claims 111 to 128, wherein said antigen-binding protein comprises a heavy chain constant region, which comprises an IgG-derived constant region.

130. The system according to claim 129, wherein said heavy chain constant region comprises a constant region derived from a protein selected from the group consisting of: IgG1, IgG2, IgG3 and IgG4.

131. The system according to any one of claims 111 to 130, wherein said antigen-binding protein comprises a light chain constant region, which comprises an Igκ-derivedconstant region or an Igλ-derived constant region.

132. The system according to claim 131, wherein said light chain constant region comprises a human Igκ-derived constant region.

133. The system according to any one of claims 111 to 132, wherein said antigen-binding protein comprises an antibody or an antigen-binding fragment thereof.

134. The system according to claim 133, wherein said antigen-binding fragment is selected from the group consisting of: Fab, Fab', F(ab)2, a Fv fragment, F(ab')2, scFv, di-scFv, VHH and/or dAb.

135. The system according to any one of claims 133 to 134, wherein said antibody is selected from the group consisting of: a monoclonal antibody, a murine antibody and a chimeric antibody.

136. The system according to any one of claims 108 to 135, wherein said detection module comprises an instrument capable of detecting a content of said DNA damage-inducible transcript 4-like DDIT4L intron retention spliced product DIR, and/or of said nucleic acid encoding said DIR.

137. The system according to any one of claims 108 to 136, wherein said detection module comprises a substance and/or an instrument capable of detecting a content of a marker associated with said cognitive impairment and/or said neurodegenerative disease.

138. The system according to claim 137, wherein said marker associated with said cognitive impairment and/or said neurodegenerative disease comprises AD7C-NTP, pTau-181, pTau-217 and /or Aβ1-42.

139. The system according to any one of claims 108 to 138, wherein said detection module outputs a content value of said DIR and/or of said nucleic acid encoding said DIR.

140. The system according to any one of claims 87 to 139, comprising a determination module capable of determining a diagnosis result and/or a disease progression result of said subject based on the content of said DIR and/or of said nucleic acid encoding said DIR as well as said normal control value and/or said early control value;
wherein said normal control value comprises a content of said DIR and/or of said nucleic acid encoding said DIR in a normal subject; and wherein said early control value comprises a previously measured content of said DIR and/or of said nucleic acid encoding said DIR in said same subject.

141. The system according to claim 140, wherein said normal subject does not suffer from said cognitive impairment and/or said neurodegenerative disease.

142. The system according to any one of claims 140 to 141, wherein when the content of said DIR and/or of said nucleic acid encoding said DIR in the sample of said subject is significantly higher than said normal control value, said determination module diagnoses said subject with said cognitive impairment and/or said neurodegenerative disease.

143. The system according to claim 140 to 142, wherein said subject has been diagnosed with said cognitive impairment and/or said neurodegenerative disease.

144. The system according to any one of claims 140 to 143, wherein when the content of said DIR and/or of said nucleic acid encoding said DIR in the sample of said subject is significantly higher than said early control value, said determination module diagnoses said subject with aggravated progression of said cognitive impairment and/or of said neurodegenerative disease.

145. The system according to any one of claims 140 to 144, wherein said determination module outputs a diagnosis result of said subject.

146. The system according to any one of claims 87 to 145, comprising a display module capable of displaying said diagnosis result of said determination module.

147. The system according to any one of claims 87 to 146, comprising a storage module capable of storing said content value output by said detection module, and/or said diagnosis result output by said determination module.

148. A medicament screening method, comprising the step of: detecting changes in a content of DIR and/or of a nucleic acid encoding said DIR in a subject after administration of a candidate medicament, wherein said medicament is for use in prevention, treatment and/or alleviation of cognitive impairment and/or a neurodegenerative disease.

149. The method according to claim 148, wherein said DIR comprises the amino acid sequence as set forth in SEQ ID NO: 1.

150. The method according to any one of claims 148 to 149, wherein said nucleic acid encoding said DIR comprises a nucleotide sequence as set forth in SEQ ID NO: 8.

151. The method according to any one of claims 148 to 150, wherein said nucleic acid encoding said DIR comprises a nucleotide sequence as set forth in SEQ ID NO: 93.

152. The use according to any one of claims 148 to 151, wherein said cognitive impairment comprises cognitive impairment caused by normal aging, Lewy body dementia LBD, frontotemporal dementia and/or vascular dementia.

153. The method according to any one of claims 148 to 152, wherein the cognitive impairment-inducing diseases comprise Alzheimer's disease, multi-infarct type, Parkinson's disease, AIDS and/or Creutzfeldt-Jakob disease CJD.

154. The method according to any one of claims 148 to 153, wherein said cognitive impairment comprises mild cognitive impairment MCI, intermediate cognitive impairment and late cognitive impairment.

155. The method according to any one of claims 148 to 154, wherein said cognitive impairment comprises multi-domain amnestic MCI aMCI-m.

156. The method according to any one of claims 148 to 155, wherein said neurodegenerative disease comprises an acute neurodegenerative disease and a chronic neurodegenerative disease.

157. The method according to any one of claims 148 to 156, wherein said neurodegenerative disease comprises a neurodegenerative disease caused by neuronal death and glial cell homeostasis, a neurodegenerative disease caused by aging, a neurodegenerative disease caused by affected CNS cell function, a neurodegenerative disease caused by abnormal intercellular communication and/or a neurodegenerative disease caused by impaired cell mobility.

158. The method according to any one of claims 148 to 157, wherein said neurodegenerative disease comprises Alzheimer's disease, Parkinson's disease, multiple sclerosis MS, amyotrophic lateral sclerosis ALS and/or Huntington's disease HD.

159. The method according to any one of claims 148 to 158, wherein said neurodegenerative disease comprises presenile dementia.

160. The method according to any one of claims 148 to 159, wherein said neurodegenerative disease comprises early presenile dementia, intermediate presenile dementia and/or late presenile dementia.

161. The method according to any one of claims 148 to 160, wherein when the content of said DIR and/or of said nucleic acid encoding said DIR in said subject is reduced due to administration of said candidate medicament, the candidate medicament has a therapeutic effect.

162. The method according to any one of claims 148 to 161, wherein said administration comprises injection.

163. The method according to any one of claims 148 to 162, wherein said medicament comprises a small molecule medicament and/or a bio-macromolecular medicament.
